# FASCICULE DE BREVET EUROPEEN

(11) **EP 2 240 449 B1**
(45) Date de publication et mention de la délivrance du brevet: **06.01.2016**
(21) Numéro de dépôt: 09710826.0
(22) Date de dépôt: 06.02.2009
(51) Int. Cl.: C07D 211/90, C07D 471/04, A61K 31/4422, A61K 31/437, A61P 31/06

(54) **NOUVEAUX DÉRIVÉS ANTI-INFECTIEUX, LEUR PROCÉDÉ DE PRÉPARATION, COMPOSITIONS PHARMACEUTIQUES LES CONTENANT ET LEURS UTILISATIONS EN THÉRAPEUTIQUE**
NEUE ANTIINFEKTIÖSE DERIVATE, VERFAHREN ZU DEREN HERSTELLUNG, PHARMAZEUTISCHE ZUSAMMENSETZUNGEN, DIE DIESE ENTHALTEN, UND ANWENDUNGEN DIESER DERIVATE BEI DER BEHANDLUNG
NOVEL ANTI-INFECTIOUS DERIVATIVES, METHOD FOR THE PRODUCTION THEREOF, PHARMACEUTICAL COMPOSITIONS CONTAINING SAME AND USES OF SAID DERIVATIVES IN TREATMENT

(30) Priorité: 07.02.2008 FR 0850778
(43) Date de publication de la demande: 20.10.2010
(73) Titulaire: Centre National de la Recherche Scientifique (CNRS), 75016 Paris (FR); Université Paul Sabatier de Toulouse III, 31062 Toulouse Cedex 9 (FR)
(72) Inventeur: BERNADOU, Jean, F-31400 Toulouse (FR); BERNARDES-GENISSON, Vania, F-31400 Toulouse (FR); DELAINE, Tamara, F-31400 Toulouse (FR); QUEMARD, Annaïck, F-31450 Montgiscard (FR)
(74) Mandataire: Blot, Philippe Robert Emile
(86) Numéro de dépôt international: PCT/FR2009/050195
(87) Numéro de publication internationale: WO 2009/101345

(56) Documents cités:
- WO-A-2008/009122
- BROUSSY S ET AL: "The first chemical synthesis of the core structure of the benzoylhydrazine-NAD adduct, a competitive inhibitor of the Mycobacterium tuberulosis enoyl reductase" J. ORG. CHEM., vol. 70, 2005, pages 10502-10510, XP002496833
- OGATA M ET AL: "SYNTHESIS AND ANTIMYCOTIC PROPERTIES OF 3-(1-IMIDAZOLYL)INDOLIN-2-ONES" EUROPEAN JOURNAL OF MEDICINAL CHEMISTRY, EDITIONS SCIENTIFIQUE ELSEVIER, PARIS, FR, vol. 16, no. 4, 1 janvier 1981 (1981-01-01), pages 373-378, XP009078474 ISSN: 0223-5234
- ABRAMOVITCH R A ET AL: "New Ring Systems from 1,2-Benzisothiazole-1,1-Dioxides and Related Compounds" TETRAHEDRON, ELSEVIER SCIENCE PUBLISHERS, AMSTERDAM, NL, vol. 52, no. 9, 26 février 1996 (1996-02-26), pages 3339-3354, XP004104357 ISSN: 0040-4020
- DATABASE BEILSTEIN BEILSTEIN INSTITUTE FOR ORGANIC CHEMISTRY, FRANKFURT-MAIN, DE; XP002496835 Database accession no. BRN: 5543587 & LIEBIGS ANNALEN DER CHEMIE, vol. 1, 1985, pages 167-182,
- DATABASE BEILSTEIN BEILSTEIN INSTITUTE FOR ORGANIC CHEMISTRY, FRANKFURT-MAIN, DE; XP002496836 Database accession no. BRN: 1623583 & CHEMICAL AND PHARMACEUTICAL BULLETIN, vol. 20, 1972, pages 2128-2131,
- DATABASE BEILSTEIN BEILSTEIN INSTITUTE FOR ORGANIC CHEMISTRY, FRANKFURT-MAIN, DE; XP002496837 Database accession no. BRN:1586486 & CHEMISCHE BERICHTE, vol. 105, 1972, pages 2933-2954,
- DATABASE BEILSTEIN BEILSTEIN INSTITUTE FOR ORGANIC CHEMISTRY, FRANKFURT-MAIN, DE; XP002496838 Database accession no. BRN: 1536983 & JOURNAL OF THE CHEMICAL SOCIETY, PERKIN TRANSACTION 1: ORGANIC AND BIO-ORGANIC CHEMISTRY, vol. 10, 1996, pages 1041-1046,
- DATABASE BEILSTEIN BEILSTEIN INSTITUTE FOR ORGANIC CHEMISTRY, FRANKFURT-MAIN, DE; XP002496839 Database accession no. BRN: 1538404 & JOURNAL OF THE CHEMICAL SOCITEY, SECTION C: ORGANIC, 1970, pages 1600-1606,
- DATABASE CAPLUS CHEMICAL ABSTRACTS SERVICE, COLUMBUS, OHIO, US; XP002496840 extrait de STN accession no. 1983:521944 Database accession no. 99:121944 & EGYPTIAN JOURNAL OF CHEMISTRY, vol. 24, no. 4-6, 1982, pages 435-443,
- DATABASE CAPLUS CHEMICAL ABSTRACTS SERVICE, COLUMBUS, OHIO, US; XP002496841 extrait de STN accession no. 1987:50081 Database accession no. 106:50081 & LATVIJAS PSR ZINATNU AKADEMIJAS VESTIS, KIMIJAS SERIJA, vol. 1, 1986, pages 19-27,
- EBERLE M K ET AL: "The preparation of 11-aryl-11H-isoindolo[2,1-a]benzimidazol-1 1-ols" J. ORG. CHEM., vol. 38, no. 22, 1973, pages 3872-3874, XP002496834

## Description

La présente invention est relative à des molécules de type inhibiteur bi-substrat associant un substituant hydrophobe à un analogue du métabolite actif de l'isoniazide, à leur procédé de préparation, aux compositions pharmaceutiques les contenant et à leur utilisation, notamment à titre d'inhibiteur d'énoyl-ACP (Acyl Carrier Protein) réductase, pour la préparation d'un médicament anti-infectieux, notamment d'un médicament antituberculeux.

Dans le cadre de la lutte contre les maladies infectieuses, le milieu médical est toujours en attente de nouvelles molécules actives pour lutter efficacement contre le problème crucial des résistances. A cet égard et à titre d'exemple, le traitement de la tuberculose, maladie en réémergence très préoccupante dans le monde depuis une vingtaine d'année, pose le problème du renouvellement des antibiotiques actuels de première ligne dont l'efficacité est très sensiblement affectée par les résistances développées par le germe responsable de cette pathologie.

L'invention repose sur la conception raisonnée de nouveaux composés basée sur une connaissance améliorée du mécanisme d'action d'un médicament de référence et des mécanismes de résistances.

Le système d'élongation des acides gras (Système Fatty Acid Synthase II ou FAS-II) est nécessaire à la biosynthèse des acides mycoliques, constituants spécifiques et composants essentiels de l'enveloppe des mycobactéries, notamment de *Mycobacterium tuberculosis* (ou bacille de Koch), agent pathogène de la tuberculose, de *M. leprae*, agent de la lèpre, et d'autres mycobactéries pathogènes opportuniste (ex : *M. avium, M. ulcerans, M. marinum*). Cette cible thérapeutique potentielle se retrouve chez certains autres agents infectieux (ex *Plasmodium falciparum*) parasite responsable du paludisme).

L'isoniazide (INH) est un antibiotique antituberculeux de première ligne dont l'efficacité est de plus en plus limitée par l'apparition de souches de *M*. *tuberculosis* résistantes. Il s'agit d'une pro-drogue qui est activée par oxydation dans la mycobactérie grâce à une catalase peroxydase (KatG) avec formation d'adduits covalents isonicotinoyl-NAD (INH-NAD) considérés comme les métabolites actifs de l'isoniazide. Ces adduits sont d'excellents inhibiteurs d'InhA, une enzyme du système FAS-II. Des adduits analogues, isonicotinoyl-NADP (INH-NADP), inhibent également une autre enzyme du système FAS-II, la protéine MabA, qui présente une structure 3D apparentée à celle d'InhA. Les phénomènes de résistance développés consistent en grande partie en des mutations qui affectent principalement l'enzyme KatG (défaut d'activation). Des mutations sont également rencontrées aussi en moindre proportion au niveau de l'enzyme cible InhA (perte d'affinité de l'adduit inhibiteur pour sa cible *in vivo*) ou de son promoteur (surproduction de la cible). La conception d'inhibiteurs bi-substrats ne nécessitant pas d'étape d'activation préalable par KatG et interagissant de façon idéale au sein du site actif d'InhA avec des régions de la protéine excluant les sites de mutation les plus fréquemment rencontrés doit permettre de contourner en grand partie les problèmes actuels de résistance à l'INH.

Les présents inventeurs ont donc mis au point des inhibiteurs d'InhA de type bi-substrat associant (i) un motif dérivé des métabolites actifs de l'isoniazide de type pyridine, pyridinium ou dihydropyridine et structures apparentées, et (ii) un substituant hydrophobe visant le site du substrat.

Sans être lié par la théorie, les composés identifiés selon la présente invention possèdent également d'autres cibles et présentent des propriétés inhibitrices sur des souches bactériennes sans équivalent d'InhA, ce qui en fait des composés anti-infectieux à large gamme d'application.

Broussy et al., J. Org. Chem. 2005, vol. 70, p. 10502-10510 décrit des composés hétérocycliques et leur utilisation pour le traitement de la tubercolose.

La présente invention concerne des composés de formule générale (I): 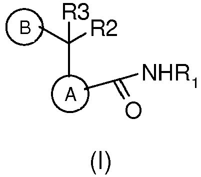 à l'état de base ou de sel d'addition à un acide, ainsi qu'à l'état d'hydrate ou de solvat pharmaceutiquement acceptables,
dans laquelle :
- le cycle Ⓐ représente un noyau aromatique ou non aromatique, de 6 chaînons, comportant éventuellement un ou plusieurs atomes d'azote, le(s) dit(s) atome(s) d'azote pouvant être éventuellement substitué(s) par
   un groupe tétrahydrofurane éventuellement substitué, tel que le 2-hydroxyméthyl-tétrahydrofurane-3,4-diol ; ou par
   un groupe -CH₂-E où E est choisi parmi -CONRR' ; -CO-SR ; phényle substitué par exemple par un groupe CN ou NO₂ ; -CO-OAlkyle ; -CO-OAlkyle-OH ; -O-Alkyle-OAc ; où R et R', identiques ou différents représentent indépendamment un atome d'hydrogène ou un groupe alkyle ;
   plus préférentiellement, l'atome d'azote est éventuellement substitué par un groupe choisi parmi les groupes -CH₂-CO-OAlkyle ; -CH₂-CO-OAlkyle-OH, -CH₂-O-Alkyle-OAc ;
   et/ou le(s) dit(s) atome(s) d'azote pouvant être présent(s) sous forme de sels de pyridinium, le contre-ion étant alors l'anion d'un atome d'halogène, tel que le bromure ou autre anion pharmaceutiquement acceptable ;

De préférence, Ⓐ est un noyau phényle, pyridine, pyrazine ou dihydropyridine éventuellement substitué.

Plus préférentiellement, Ⓐ représente un groupe dihydropyridine ou pyridine, éventuellement substitué par un groupe choisi parmi les groupes -CH₂-CO-OAlkyle, -CH₂-CO-OAlkyle-OH, -CH₂-O-Alkyle-OAC, et/ou éventuellement sous forme d'halogénure de pyridinium ;
- Ⓑ représente un groupe aryle ou hétéroaryle, mono ou bicyclique, de 5 à 10 chaînons, substitué par un ou plusieurs groupes choisis parmi les groupes ; -(C₅-C₂₀)Alkyle ; -O(C₂-C₂₀)Alkyle ; -S(O)ₚAlkyle où p=0, 1 ou 2 ; Alkényle ; Alkynyle.

De préférence, Ⓑ est un noyau phényle substitué par un ou plusieurs groupes choisis parmi les groupes -OH, -(C₅-C₂₀)Alkyle ; -O(C₅-C₂₀)Alkyle ; -S(O)ₚ(C₅-C₂₀)Alkyle où p=0, 1 ou 2 ; (C₅-C₂₀)Alkényle ; (C₅-C₂₀)Alkynyle ; -O(C₅-C₂₀)Alkényle ; -C(=)O-(C₅-C₂₀)Alkyle ; -C(=O)-(C₅-C₂₀)Alkényle ; phényle substitué par un groupe Alkyle (C₅-C₂₀); Cycloalkyle éventuellement substitué par un groupe Alkyle.

Plus préférentiellement, Ⓑ est un noyau phényle substitué par un groupe -OH, -(C₅-C₂₀)Alkyle, O(C₅-C₂₀)Alkényle, O(C₅-C₂₀)Alkyle, -S(O)ₚAlkyle où p=0, 1 ou 2.

Plus préférentiellement, Ⓑ est substitué en position ortho ou méta ;
- R1 représente un atome d'hydrogène ou un groupe Alkyle et R2 et R3 forment ensemble un groupe =O ;
ou, alternativement,
R3 représente un groupe -OH ou O-alkyle et R2 forme avec R1 une liaison simple liant l'atome d'azote à l'atome de carbone substitué par R3, Ⓐ et Ⓑ, de façon à former un noyau indanone par fusion au cycle Ⓐ.

De préférence, R3=OH ou -OAlkyle et R1 et R2 forment ensemble une simple liaison, ou
R3 et R2 forment un groupe =O et R1 représente un atome d'hydrogène.

Dans ce qui précède et ce qui suit, il est entendu que les groupes -C(=O)NHR1 et C(R3)(R2)- Ⓑ sont situés sur deux positions adjacentes du noyau Ⓐ.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- atome d'halogène : un fluor, un chlore, un brome ou un iode ;
- groupe alkyle : un groupe aliphatique saturé linéaire ou ramifié de 1 à 20 atomes de carbone, sauf mention contraire. A titre d'exemples, on peut citer les groupes méthyle, éthyle, propyle, isopropyle, butyle, isobutyle, tertbutyle ;
- groupe cycloalkyle : un groupe alkyle cyclique de 3 à 10 atomes de carbone. A titre d'exemples, on peut citer les groupes cyclopropyle, méthylcyclopropyle, cyclobutyle, cyclopentyle, cyclohexyle, etc ;
- groupe alkényle : un groupe aliphatique de 2 à 20 atomes de carbone mono- ou poly-insaturé, linéaire ou ramifié, comprenant par exemple une ou deux insaturations éthyléniques;
- groupe alkynyle : un groupe aliphatique de 2 à 20 atomes de carbone mono- ou poly-insaturé, linéaire ou ramifié, comprenant par exemple une ou deux insaturations acétyléniques;
- groupe aryle : un groupe aromatique cyclique comprenant entre 5 et 10 atomes de carbone. A titre d'exemples de groupes aryles, on peut citer un phényle ou naphthyle;
- groupe hétéroaryle : un groupe aromatique cyclique comprenant entre 5 et 10 atomes de carbone et comprenant entre 1 et 3 hétéroatomes, tels que l'azote, l'oxygène ou le soufre. A titre d'exemples de groupes hétéroaryles, on peut citer notamment la pyridine.

Particulièrement, on préfère les composés de formule (I) dans laquelle :
Ⓐ représente un groupe dihydropyridine, éventuellement substitué par un groupe choisi parmi -CH₂COOAlkyle, -CH₂COOAlkyleOH ;
Ⓑ représente un groupe phényle, substitué en position ortho ou méta, de préférence méta, par un groupe choisi parmi les groupes (C₅-C₂₀) alkyle, -O(C₅-C₂₀)Alkyle, -S(O)p Alkyle où p=0,1 ou 2, phényle éventuellement substitué par Alkyle
R1 et R2 forment ensemble une liaison simple et R3=OH, ou R1=H et R2 et R3 forment ensemble un groupe =O,
à l'état de base ou de sel d'addition à un acide, ainsi qu'à l'état d'hydrate ou de solvat pharmaceutiquement acceptables.

Parmi les composés de formule (I) objets de l'invention, on peut notamment citer les composés suivants :
- 1-(3-dodécylphényl)-1-hydroxy-1,2-dihydropyrrolo[3,4-c]pyridin-3-one ;
- 1-(3-dodécyloxyphényl)-1-hydroxy-1,2-dihydropyrrolo[3,4-c]pyridin-3-one ;
- bromure de 1-(3-dodécylphényl)-5-[2-(éthyloxy)-2-oxoéthyl]-1-hydroxy-3-oxo-1,2-dihydropyrrolo[3,4-c]pyridinium;
- 3-aminocarbonyl-[4-(3-dodécylbenzoyl)-1,4-dihydro-pyridin-1-yl] acétate d'éthyle ;
- 1-(3-octyloxyphényl)-1-hydroxy-1,2-dihydro-3*H*-pyrrolo[3,4-*c*]pyridin-3-one ;
- 1-(2-dodécyloxyphényl)-1-hydroxy-1,2-dihydro-3*H*-pyrrolo[3,4-*c*]pyridin-3-one ;
- 1-[3-(dodécylthio)phényl]-1-hydroxy-1,2-dihydro-3*H*-pyrrolo[3,4-*c*]pyridin-3-one ;
- 1-(3-dodécyloxyphényl)-1-méthoxy-1,2-dihydro-3*H*-pyrrolo[3,4-*c*]pyridin-3-one ;
- 1-Hydroxy-1-(3-(propèn-3-yl)oxyphényl)-1,2-dihydropyrrolo[3,4-*c*]pyridin-3-one ;
- 1-Hydroxy-1-(3-hydroxyphényl)-1,2-dihydropyrrolo[3,4-*c*]pyridin-3-one ;
- 1-Hydroxy-1-(3-octylphényl)-1,2-dihydropyrrolo[3,4-*c*]pyridin-3-one ;
- 1-Hydroxy-1-(3-(4-nonylphényl)phényl)-1,2-dihydropyrrolo[3,4-*c*]pyridin-3-one ;
- bromure de 1-[3-(dodécyloxy)phényl]-5-(2-éthoxy-2-oxoéthyl)-1hydroxy-3-oxo-2,3-dihydro-1*H*-pyrrolo[3,4-*c*] pyridin-5-ium ;
- bromure de 1-[(3-(dodécyloxy)phényl)-1-hydroxy-5-[2-(3-hydroxypropoxy)-2-oxyéthyl]-3-oxo-2,3-dihydro-1*H*-pyrrolo[3,4-*c*]pyridin-5-ium ;
- 3-aminocarbonyl-[4-(3-dodécyloxybenzoyl)-1,4-dihydropyridin-1-yl]acétate d'éthyle ;
- 3-aminocarbonyl-[4-(3-dodécyloxybenzoyl)-1,4-dihydropyridin-1-yl]acétate de 3-hydroxypropyle ;
à l'état de base ou de sel d'addition à un acide, ainsi qu'à l'état d'hydrate ou de solvat pharmaceutiquement acceptables.

Selon un autre objet, la présente demande concerne également le procédé de préparation des composés de formule générale (I).

Conformément à l'invention, on peut préparer les composés de formule générale (I) selon le procédé qui suit.

Plus précisément, selon un premier mode de réalisation, les composés de formule générale (I) dans laquelle R1 et R2 forment ensemble une liaison simple et R3 représente -OH peuvent être préparés par couplage d'un composé de formule générale (II) : 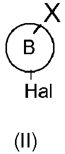 et d'un composé de formule générale (III) : 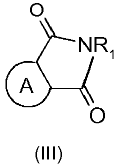 conduisant au composé de formule (IV) : 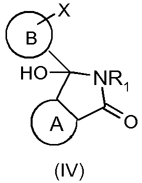 dans lesquelles Ⓐ, Ⓑ et R1 sont définis tel qu'en formule générale (I), Hal représente un atome d'halogène et X représente soit un atome d'hydrogène lorsque Ⓑ n'est pas substitué, soit le substituant de Ⓑ correspondant à la formule générale (I) désirée (dans ces deux cas, le composé (IV) correspond au composé (I)), soit un atome d'halogène le couplage étant suivi dans ce cas de la réaction de substitution de l'atome d'halogène du composé (IV) par le substituant de Ⓑ approprié correspondant à la formule générale (I) désirée.

Généralement, la réaction de couplage est conduite en présence d'une base organique ou minérale telle que *n*-BuLi ou *t*-BuLi, dans un solvant tel que le THF, à température comprise entre -78° et la température ambiante, sous atmosphère inerte.

Généralement, la réaction de substitution est conduite par application ou adaptation des réactions de substitution connues de l'homme du métier, au moyen de réactifs appropriés. Ces réactions de substitution sont par exemple décrites dans March's *Advanced Organic Chemistry,* 5^{rd} Ed., John Wiley and Sons, Inc. ou Larock, *Comprehensive Organic Transformations,* VCH Ed. A titre représentatif, par exemple, on peut citer la réaction de substitution conduisant au composé de formule générale (I) dans laquelle Ⓑ est substitué par un groupe alkyle. Cette réaction peut notamment être réalisée au moyen d'acide alkylboronique, en présence de chlorure de diphénylphosphine ferrocène de palladium (II), d'oxyde d'argent et de carbonate de potassium.

Le composé de formule générale (II) est commercial ou peut être préparé par application ou adaptation de méthodes connues pour substituer le noyau Ⓑ tel que désiré pour obtenir le composé de formule (I) dans lequel Ⓑ est substitué. A titre représentatif, le noyau Ⓑ peut être substitué par un groupe OAlkyle au moyen du produit de départ correspondant (II) dans lequel Ⓑ est substitué par un groupe hydroxy, par réaction d'un composé de type halogénure d'alkyle, en présence d'une base telle que le carbonate de potassium.

Selon un second mode de réalisation, les composés de formule générale (I) dans laquelle R1 et R2 forment ensemble une liaison simple et R3 représente -OH peuvent être préparés par cyclisation de composés de formule générale (V) : 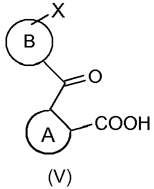 dans laquelle Ⓐ, Ⓑ et Hal sont définis comme en formule générale (IV) en présence de NHR1 (V'),
conduisant au composé de formule (IV) : 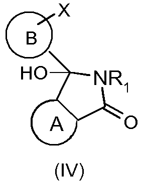 dans lesquelles R1 est défins tel qu'en formule générale (I), et X représente soit un atome d'hydrogène lorsque Ⓑ n'est pas substitué, soit le substituant de Ⓑ correspondant à la formule générale (I) désirée (dans ces deux cas, le composé (IV) correspond au composé (I)), soit un atome d'halogène la cyclisation étant alors suivie dans ce cas de la réaction de substitution de l'atome d'halogène du composé (IV) par le substituant de Ⓑ approprié correspondant à la formule générale (I) désirée.

Généralement, la réaction de cyclisation est réalisée au moyen de chlorure de thionyle pour former de chlorure d'acyle correspondant suivi de l'ajout d'un composé (V') de formule :

NHR1 (V')

Généralement, cette réaction est réalisée, sans isolement du chlorure d'acyle intermédiaire, à température comprise entre la température ambiante et la température d'ébullition du mélange réactionnel.

A titre de composé de formule (V'), on peut notamment citer l'ammoniaque en solution dans l'eau.

Généralement, la réaction de substitution est réalisée comme discuté ci-avant.

Le composé de formule (V) peut être obtenu à partir d'un composé correspondant de formule (VI) : 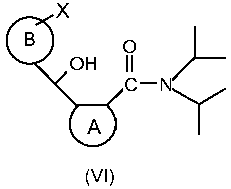 dans laquelle Ⓐ, Ⓑ et X sont définis comme en formule générale (V). Cette réaction est généralement réalisée en milieu acide, par exemple dans l'acide formique, à température comprise entre la température ambiante et la température de reflux du mélange réactionnel.

Le composé de formule (VI) peut être obtenu par réduction du composé correspondant de formule (VII) : 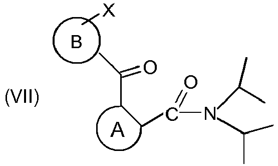 dans laquelle Ⓐ, Ⓑ et X sont définis comme en formule (VI). Généralement, cette réaction peut être réalisée au moyen d'un réducteur tel que le tétraborohydrure de sodium, dans un solvant tel qu'un alcool, par exemple l'éthanol.

Le composé de formule (VII) peut être préparé par couplage des composés de formule (VIII) et (IX) correspondants : 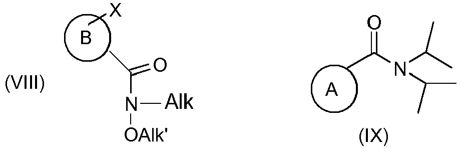 dans lesquelles Ⓐ, Ⓑ et X sont tels que définis en formule (VII) et Alk, Alk', identiques ou différents, représentent indépendamment un groupe alkyle. Généralement, cette réaction est réalisée en présence d'une base telle qu'un composé à base de lithium, par exemple le lithium diisopropylamide, dans un solvant organique approprié, tel que l'éther diéthylique. Préférentiellement, cette réaction est conduite en milieu anhydre, sous atmosphère inerte, par ajout de la base au composé de formule (IX) puis ajout du composé de formule générale (VIII). Préférentiellement, cette réaction est réalisée à température comprise entre -75°C et 0°C.

Le composé de formule (VIII) peut être obtenu par couplage des composés de formule (X) et (XI) correspondants : 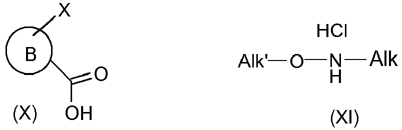 dans lesquelles Ⓑ, X, Alk et Alk' sont définis comme en formule générale (VIII). Généralement, cette réaction est conduite au moyen de réactifs de couplage de type EDCI tel que 1-(3-diméthylaminopropyle)-3-éthylcarbodiimide (sel de chlorhydrate) et HOBT (1-hydroxy-*1H*-benzotriazole hydraté) et en présence d'une base telle que la triéthylamine.

Lorsque Ⓐ représente un noyau pyridinium dans lequel l'atome d'azote est quaternarisé, le procédé de préparation comprend en outre l'étape consistant à quaternariser le composé de formule générale (I) dans lequel Ⓐ représente un noyau pyridine au moyen d'un composé de formule générale (XII) :

R-Hal (XII)

dans laquelle Hal représente un atome d'halogène, tel que le brome et R représente un groupe de type -CH₂E, E étant un groupe électroattracteur défini comme en formule générale (I). Généralement, cette réaction est conduite dans un solvant tel que le tétrahydrofurane anhydre, à température comprise entre la température ambiante et la température de reflux du mélange réactionnel.

Lorsque Ⓐ représente un noyau dihydropyridine et que R2 et R3 forment ensemble un groupe =O et R1 représente un atome d'hydrogène, le composé de formule générale (I) peut être obtenu par réduction à partir du composé de formule générale (I) dans laquelle Ⓐ représente un noyau pyridinium. A titre de réducteur, on peut notamment citer le triacétoxyborohydrure de sodium en milieu acétique. Généralement, cette réaction est conduite à température comprise entre - 10°C et la température ambiante.

Le schéma I ci-dessous illustre le procédé selon l'invention.

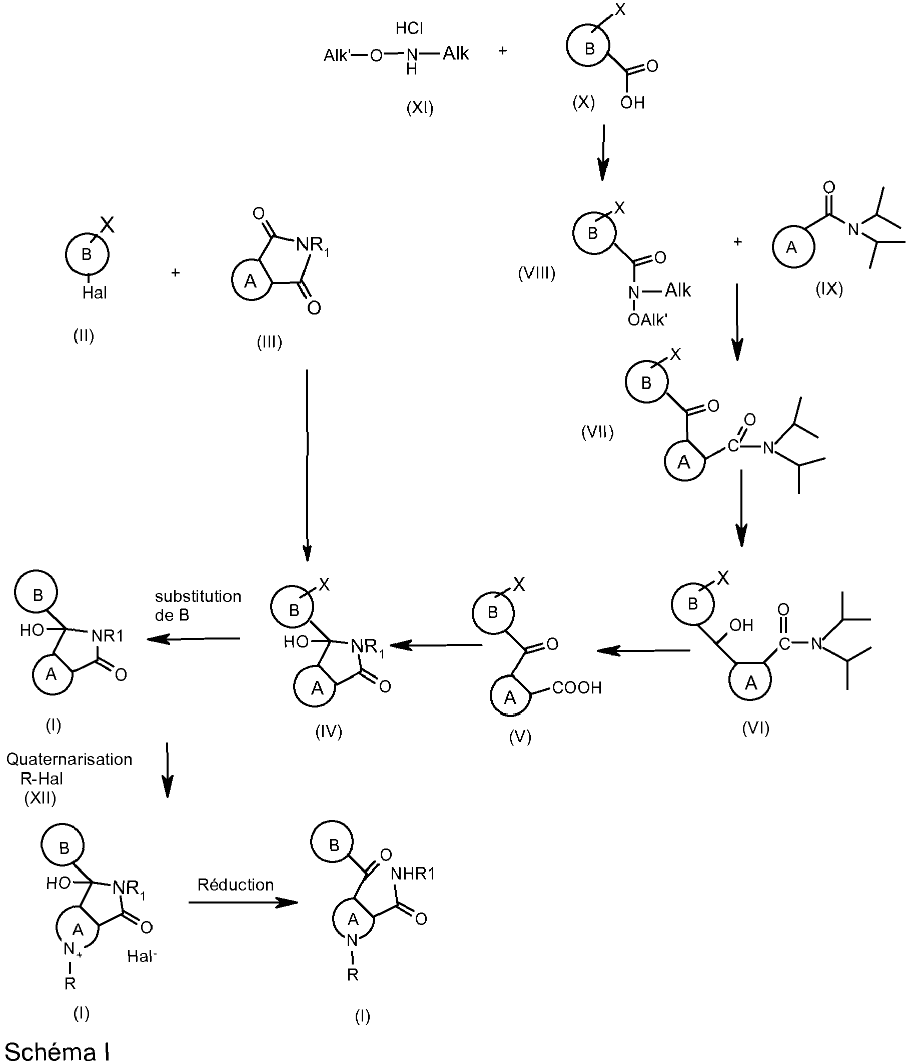

Eventuellement, le procédé selon l'invention comprend l'étape ultérieure consistant à isoler le produit désiré obtenu.

Les composés de départ (II), (III), (IX), (X), (XI) et les réactifs appropriés sont disponibles commercialement ou peuvent être préparés par application ou adaptation de méthodes connues de l'homme du métier.

Les composés selon l'invention présentent des propriétés anti-infectieuses remarquables.

Ainsi, selon un autre de ses aspects, l'invention a pour objet des médicaments qui comprennent un composé de formule (I) tel que défini ci-avant et au moins un véhicule pharmaceutiquement acceptable.

Ainsi, les composés selon l'invention peuvent être utilisés, chez l'homme ou chez l'animal comme anti-infectieux, notamment dans le traitement ou la prévention de :
- mycobactérioses, plus particulièrement la tuberculose, la lèpre ou les maladies opportunistes, telles que celles liées à *Mycobacterium avium, M. bovis, M. marinum* et/ou *M. ulcerans*, ;
- le paludisme ;
- toute infection liée à un agent pathogène possédant une enzyme de type enoyl-Acyl Carrier Protein reductase, ou une enzyme de structure apparentée, appartenant à la superfamille des Short Chain Dehydrogenase Reductases (SDR).

Plus préférentiellement, les composés selon l'invention peuvent être utilisés dans le traitement de la tuberculose.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Les compositions pharmaceutiques selon l'invention peuvent comprendre, à titre de principe actif, de 10 à 800 mg d'un composé de formule générale (I) selon l'invention.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 224 mg |
| Croscaramellose sodique | 6,0 mg |
| Amidon de maïs | 15,0 mg |
| Hydroxypropyl-méthylcellulose | 2 mg |

| | |
|---|---|
| Stéarate de magnésium | 3,0 mg |

La dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

Selon un autre objet, la présente invention concerne également les combinaisons d'un composé de formule générale (I) selon l'invention et un principe actif à activité anti-infectieuse. A titre de composé anti-infectieux, on peut notamment citer l'isoniazide, rifampicine, pyrazinamide, ethambutol, chloroquine, ou tout autre molécule antibiotique utilisée actuellement en clinique.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

Tous les solvants utilisés sont de pureté "reagent grade" ou "HPLC grade".

La présente invention a également pour objet le procédé de préparation des molécules de formule générale (I) précitées.

Selon un autre objet, la présente invention concerne également des composés de formule générale (I) pour l'utilisation en thérapeutique.

Les exemples suivants sont donnés à titre illustratif et non limitatif de la présente invention.

### PREPARATION 1 : synthèse du composé 1 de formule

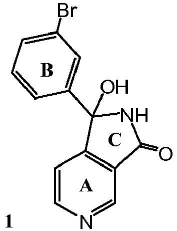

### 1^{re} étape : synthèse d'un composé (1a)

Le composé **1a** répond à la formule suivante :

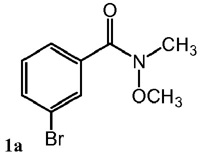

A une suspension d'acide 3-bromobenzoïque (4,0 g, 20,0 mmol), de chlorhydrate de *N,O*-diméthylhydroxylamine (2,0 g, 21,0 mmol), de 1-hydroxy-1*H-*benzotriazole hydraté (920 mg, 6,0 mmol) et de chlorhydrate de 1-(3-diméthylaminopropyl)-3-éthylcarbodiimide (4,6 g, 24,0 mmol) dans l'acétonitrile anhydre (25 mL) est additionnée goutte à goutte, sous atmosphère inerte, la triéthylamine (2,9 mL, 19,3 mmol). Après 2 h sous agitation à température ambiante, 20 mL d'eau distillée sont ajoutés au mélange réactionnel puis le solvant est évaporé sous pression réduite. Le résidu est repris dans l'eau et extrait à l'acétate d'éthyle (3 x 20 mL). Les phases organiques sont réunies, séchées sur sulfate de sodium anhydre et concentrées à l'évaporateur rotatif pour donner 4,7 g (98%) d'amide **1a** sous la forme d'une huile jaune pâle.

**IR (film, cm⁻¹) :** 3066, 2969, 2934, 1644, 1383, 1211, 662. **RMN ¹H (250 MHz, CDCl₃)** δ (ppm) : 7,80 (t, 2H), 7,61-7,54 (m, 2H), 7,26 (t, 1 H), 3,53 (s, 3H), 3,34 (s, 3H). **RMN ¹³C (50 MHz, CDCl₃)** δ(ppm) : 168,0, 135,6, 133,4, 131,0, 129,4, 126,6, 121,8, 61,0, 33,4. **SMHR (FAB)** pour C₉H₁₁NO₂Br : observ. 243,9969, théor. 243,9973.

### 2^{e} étape : synthèse d'un composé (1b) à noyau diisopropylnicotinamide comportant en position 4 un résidu 3-bromobenzoyle

Le composé **1 b** répond à la formule suivante :

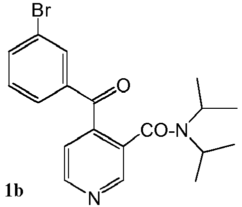

A une solution de *N,N-*diisopropylnicotinamide (618 mg, 3,0 mmol) dans l'éther diéthylique anhydre (75 mL) est ajouté goutte à goutte, à -78°C, sous atmosphère inerte, le diisopropylamidure de lithium (2,7 mL, 5,4 mmol, 2M en solution dans THF/*n*-Heptane). Après 20 minutes sous agitation à -50°C, une solution de l'amide de Weinreb **1a** (1,1 g, 4,5 mmol) dans l'éther diéthylique anhydre (8 mL) est additionnée goutte à goutte, à -78°C, en 8 fois toutes les 10 minutes. Le milieu réactionnel est laissé revenir à température ambiante sous agitation et la réaction est suivie par CCM (éluant : dichorométhane/méthanol : 95/5). Puis 50 mL d'eau distillée sont ajoutés à la solution et le mélange réactionnel est extrait à l'acétate d'éthyle (4 x 20 mL). Les phases organiques sont réunies, lavées avec une solution saturée en chlorure de sodium, séchées sur sulfate de sodium anhydre et concentrées à l'évaporateur rotatif. Le résidu huileux obtenu est purifié par chromatographie sur colonne de gel de silice (éluant : gradient dichlorométhane/méthanol de 100/0 à 98/2), pour donner 330 mg (28%) du céto-amide **1** b sous forme d'un solide jaune.

**P_{f}** : 112°C. **IR (KBr, cm⁻¹)** : 3061, 2971, 2933, 1675, 1630, 1343, 1267, 668. **RMN ¹H (250 MHz, CDCl₃)** δ (ppm) : 8,71 (d, 1 H), 8,63 (s, 1 H), 7,94 (t, 1 H), 7,73-7,66 (m, 2H), 7,32 (t, 1 H), 7,30 (d, 1 H), 3,87-3,77 (m, 1 H), 3,52-3,41 (m, 1 H), 1,38 (d, 6H), 1,22 (d, 6H). **RMN ¹³C (63 MHz, CDCl₃)** δ(ppm) : 193,5, 166,4, 149,4, 146,9, 143,6, 137,4, 133,6, 122,8, 136,5, 132,6, 130,1, 128,8, 122,2, 51,7, 42,4, 20,5, 20,0. **SMHR (ESI)** pour C₁₉H₂₂N₂O₂Br : observ. 389,0864 ; théor. 389,0865.

### 3^{e} étape : réduction de la fonction carbonyle de l'amidocétone 1b obtenue à la 2^{e} étape.

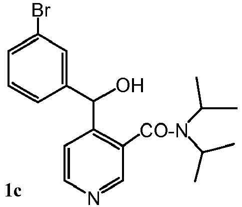

A une solution de céto-amide **1b** (627 mg, 1,6 mmol) dans l'éthanol absolu (80 mL) est ajouté le tétraborohydrure de sodium (421 mg, 8,8 mmol). Après 3 h sous agitation à température ambiante, 50 mL d'eau distillée sont ajoutés. Le mélange réactionnel est extrait au dichlorométhane (3 x 20 mL). Les phases organiques sont réunies, séchées sur sulfate de sodium anhydre et concentrées à l'évaporateur rotatif pour donner 631 mg (85%) des deux rotamères de l'hydroxy-amide **1c** sous forme d'une pâte jaune.

Les deux rotamères sont obtenus en proportion 65/35, mais pour des raisons de commodité les spectres ¹H et ¹³C sont décrits comme si les protons ou carbones du rotamère minoritaire avait la même intensité que les protons ou carbones correspondant au rotamère majoritaire.

**IR (KBr, cm⁻¹)** : 3243, 2973, 2934, 1615, 1443, 1344, 671. **RMN ¹H (250 MHz, CDCl₃)** δ (ppm) : 8,66 (d, 1 H), 8,56 (d, 1 H), 8,44 (s, 1 H), 8,40 (s, 1 H), 7,45-7,43 (m, 4H), 7,36 (d, 2H), 7,29-7,10 (m, 4H), 6,02 (s, 1 H), 5,98 (s, 1 H), 5,66 (s, 1 H), 5,62 (s, 1 H), 3,54-3,40 (m, 2H), 3,35-3,24 (m, 2H), 1,53 (d, 6H), 1,41 (d, 3H), 1,27 (d, 3H), 1,20 (d, 3H), 1,14 (d, 3H), 0,79 (d, 3H), 0,49 (d, 3H). **RMN ¹³C (63 MHz, CDCl₃)** δ(ppm) : 169,1, 168,1, 150,8, 150,4, 147,5, 145,6, 145,0, 143,5, 134,9, 134,2, 131,9, 131,8, 126,0, 125,5, 131,2, 130,9, 130,3, 130,0, 129,1, 126,5, 125,1, 124,8, 122,8, 121,8, 75,1, 70,7, 51,5, 51,4, 46,6, 46,4, 20,7, 20,6, 20,5, 20,2, 20,1, 20,0. **SMHR (FAB)** pour C₁₉H₂₄N₂O₂Br : observ. 391,1017 ; théor. 391,1021.

### 4^{e} étape : Hydrolyse de la fonction amide et oxydation de la fonction alcool de l'hydroxy-amide 1c obtenu à la 3^{e} étape pour obtention du composé 1d.

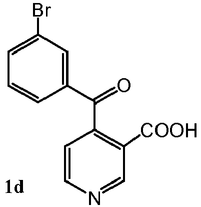

Une solution de l'hydroxy-amide **1c** (445 mg, 1,2 mmol) dans l'acide formique (85,0 mL, 2,2 mol) est chauffée à reflux pendant 24 h. Le solvant est évaporé sous pression réduite, le résidu est repris dans 25 mL d'eau distillée puis extrait à l'acétate d'éthyle (3 x 20 mL). Les phases organiques sont réunies, lavées par une solution saturée en bicarbonate de sodium (2 x 20 mL), séchées sur sulfate de sodium anhydre, puis concentrées à l'évaporateur rotatif. L'huile obtenue est reprise dans l'ammoniaque méthanolique 7 N (85 mL, 3,8 mol). Le mélange réactionnel est agité à l'air à température ambiante pendant 3 jours, puis il est concentré sous pression réduite. Le résidu est dissous dans le méthanol (30 mL) et de l'acide chlorhydrique 2 M est ajouté goutte à goutte jusqu'à pH = 1. Le mélange réactionnel est concentré à l'évaporateur rotatif. Le résidu obtenu est purifié par chromatographie sur colonne de gel de silice (éluant : gradient dichlorométhane/méthanol : de 100/0 à 80/20), pour donner 312 mg (85%) du céto-acide **1d** sous forme d'un solide jaune.

**P_{f}** : 222°C. **IR (KBr, cm⁻¹)** : 3419, 3067, 2923, 2852, 1703, 1679, 1605, 1260, 669. **RMN ¹H (300 MHz, DMSO-*d6*)** δ (ppm) : 9,11 (s, 1H), 8,68 (d, 1H), 8,25 (s, 1 H), 7,75 (d, 1 H), 7,68 (s, 1 H), 7,49 (d, 1H,), 7,41 (t, 1 H), 7,26 (d, 1 H). **RMN ¹³C (75 MHz, DMSO-*d6*)** δ (ppm): 194,8, 167,4, 151,7, 150,9, 148,1, 139,8, 132,1, 122,2, 135,4, 131,2, 130,9, 128,0, 120,7. **SMHR (ESI)** pour C₁₃H₇NO₃Br : observ. 303,9624 ; théor. 303,9609.

### 5^{e} étape : synthèse du composé 1 par cyclisation du composé 1d obtenu à la 3^{e} étape.

Une solution de céto-acide **1d** (331 mg, 1,1 mmol) dans le chlorure de thionyle (20,0 mL, 300,0 mmol) est agitée à 60°C sous atmosphère inerte pendant 2 h. Le milieu réactionnel est ensuite concentré sous pression réduite, le résidu est repris dans le dichlorométhane et est de nouveau évaporé. Cette opération est répétée deux fois. Le résidu est finalement repris dans l'acétone (17 mL) puis une solution aqueuse d'hydroxyde d'ammonium à 32% (9,2 mL, 3,0 mmol) est ajoutée goutte à goutte. La solution résultante est agitée à température ambiante pendant 1 h. Le milieu réactionnel est concentré à l'évaporateur rotatif. Le résidu obtenu est purifié par chromatographie sur colonne de gel de silice (éluant : gradient dichlorométhane/méthanol : de 100/0 à 95/5) pour donner 268 mg (80%) de l'hémiamidal **1** sous forme d'une poudre beige.

**P_{f}** : 211°C. **IR (KBr, cm⁻¹)** : 3296, 3059, 1693, 1609, 1444, 1291, 647. **RMN ¹H (500 MHz, DMSO-*d6*)** δ (ppm) : 9,53 (s, 1 H), 8,89 (d, 1 H), 8,75 (d, 1 H), 7,70 (t, 1 H), 7,56 (ddd, 1 H), 7,44 (m, 2H), 7,34 (t, 1 H), 7,33 (s, 1 H). **RMN ¹³C (126 MHz, DMSO-*d6*)** δ(ppm) : 167,5, 158,3, 153,6, 145,2, 143,8, 122,2, 131,7, 131,2, 128,8, 126,5, 125,3, 118,3, 86,6. **SMHR (FAB)** pour C₁₃H₁₀N₂O₂Br : observ. 304,9927 ; théor. 304,9926.

### EXEMPLE 1 : synthèse du composé 2 de formule :

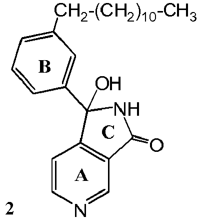

A partir du composé **1** obtenu tel que décrit selon la Préparation **1** ci-dessus, on procède à la réaction d'alkylation.

A une solution de l'hémiamidal **1** (400 mg, 1,32 mmol) dans le tétrahydrofurane anhydre (24 mL) est ajouté sous atmosphère inerte l'acide *n*-dodécyl boronique (308 mg, 1,44 mmol), le chlorure de diphénylphosphine férrocène de palladium (II) (192 mg, 0,26 mmol), l'oxyde d'argent (I) (760 mg, 3,20 mmol) et le carbonate de potassium (542 mg, 4,00 mmol). Le tube est bouché hermétiquement et chauffé à 80°C pendant 48 h. La solution est diluée dans le dichlorométhane (20 mL) puis est ajouté une solution de H₂O₂ (30%)/NaOH (10%). Laisser 2 h sous agitation. Le mélange réactionnel est extrait au dichlorométhane (3 x 50 mL). Les phases organiques sont réunies, séchées sur sulfate de sodium anhydre et concentrées sous pression réduite. Le résidu obtenu est purifié par chromatographie sur colonne de gel de silice (éluant : gradient dichlorométhane/méthanol : de 100/0 à 90/10) pour donner 125 mg (24%) de l'hémiamidal **2** sous forme d'une poudre orangée.

**P_{f}** : 108°C. **IR (KBr, cm⁻¹)** : 3478, 3413, 3064, 2925, 2851, 1706, 1615, 1280. **RMN ¹H (500 MHz, CDCl₃)** δ (ppm) : 8,82 (s, 1 H), 8,62 (d, 1 H), 7,40 (s, 1 H), 7,37-7,33 (m, 2H), 7,31-7,28 (m, 2H), 7,19 (d, 1 H), 5,43 (s large, 1 H), 2,60 (t, 2H), 1,59 (q, 2H), 1,30-1,27 (m, 18H), 0,90 (t, 3H). **RMN ¹³C (126 MHz, CDCl₃)** δ(ppm) : 168,1, 158,3, 153,2, 145,4, 144,0, 138,2, 129,3, 128,8, 125,6, 125,2, 122,7, 117,9, 88,1, 36,0, 31,5, 32,0, 29,9, 29,8, 29,7, 29,6, 29,5, 29,4, 29,3, 22,7, 14,1. **SMHR (FAB)** pour C₂₅H₃₅N₂O₂ : observ. 395,2712 ; théor. 395,2699.

### EXEMPLE 2 : synthèse du composé 3 de formule :

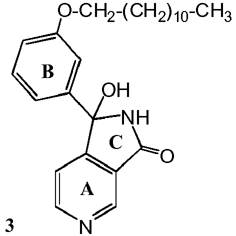

1^{re} étape : synthèse du composé (**3a**)

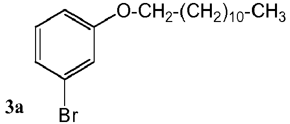

A une solution de 3-bromophénol (1,00 g, 5,78 mmol) dans le diméthylformamide anhydre (60 mL) est ajouté sous atmosphère inerte, à température ambiante, le carbonate de potassium (1,20 g, 8,70 mmol). Après 5 minutes sous agitation, le iodododécane (2,57 g, 8,70 mmol) est rajouté. Après 18 h, le mélange réactionnel est filtré. Le filtrat est dilué dans l'eau (15 mL) et extrait à l'acétate d'éthyle (3 x 30 mL). Les phases organiques sont réunies, lavées avec une solution saturée en chlorure de sodium (50 mL), séchées sur sulfate de sodium anhydre et concentrées sous pression réduite. Le résidu obtenu est purifié par chromatographie sur colonne de gel de silice (éluant : hexane) pour donner 1,86 g (94%) du bromo éther **3a** sous forme d'une huile incolore.

**IR (film, cm⁻¹)** : 3067, 2924, 2853, 1590, 1573, 1467, 1228, 680. **RMN ¹H (250 MHz, CDCl₃)** δ (ppm) : 7,19-7,07 (m, 3H), 6,85 (dd, 1 H), 3,96 (t, 1 H), 1,78 (q, 2H), 1,47-1,30 (m, 18H), 0,91 (t, 3H). **RMN ¹³C (63 MHz, CDCl₃)** δ(ppm) : 160,0, 130,5, 123,5, 117,7, 113,6, 122,8, 68,3, 31,9, 29,7-29,1, 26,0, 22,7, 14,1. **SM (DCI/NH₃)** *m*/*z* : 358-360 (M + NH₄⁺), 340-342 (M⁺).

### 2^{e} étape : synthèse du composé 3 par condensation de la 3.4-pyridinedicarboximide et du composé 3a obtenu à la 1^{re} étape.

A une solution de bromo éther **3a** (1,90 g, 5,57 mmol) dans le tétrahydrofurane anhydre (5,5 mL) est ajouté goutte à goutte, sous atmosphère inerte et à -78°C le *tert*-butyl lithium (7 mL, 10,5 mmol, 1,5 M dans le pentane). Après 40 minutes sous agitation à -78°C, cette solution est ajouté goutte à goutte, sous atmosphère inerte et à -78°C à une solution de 3,4-pyridinedicarboximide (550 mg, 3,7 mmol) dans le tétrahydrofurane (22 mL). Le mélange réactionnel est laissé remonter à température ambiante sous agitation. 15 mL d'eau sont rajoutés et le mélange réactionnel est extrait à l'acétate d'éthyle (3 x 25 mL). Les phases organiques sont réunies, lavées avec une solution aqueuse saturée en chlorure de sodium (40 mL), séchées sur sulfate de sodium anhydre et concentrées sous pression réduite. Le résidu huileux obtenu est purifié par chromatographie sur colonne de gel de silice (éluant : gradient dichlorométhane/méthanol : de 100/0 à 90/10) pour donner 346 mg (23%), d'un mélange para/méta (3/1) de l'hémiamidal **3**. Le mélange est recristallisé dans l'acétone pour donner **3** sous forme d'une poudre blanche (124 mg, 8%).

**P_{f}** : 131°C. **IR (KBr, cm⁻¹)** : 3413, 3201, 3066, 2923, 2853, 1718, 1615, 1260. **RMN ¹H (500 MHz, DMSO-d*6*)** δ (ppm) : 9,46 (s, 1H), 8,86 (d, 1H), 8,72 (d, 1H), 7,42 (dd, 1 H), 7,26 (t, 1 H), 7,15 (s, 1H,), 7,06 (t, 1 H), 6,98 (dd, 1 H), 6,89 (dd, 1 H), 3,94 (t, 2H), 1,69 (q, 2H), 1,39 (m, 2H), 1,30-1,25 (m, 16H), 0,86 (t, 3H). **RMN ¹³C (126 MHz, DMSO-d*6*)** δ(ppm) : 167,5, 159,1, 158,8, 153,4, 145,0, 142,7, 126,5, 131,9, 118,3, 118,0, 114,5, 112,4, 87,6, 67,9, 31,7, 29,5-29,1, 22,5, 26,0, 14,4. **SMHR (ESI)** pour C₂₅H₃₅N₂O₃ : observ. 411,2653 ; théor. 411,2648.

### EXEMPLE 3 : synthèse du composé 4 de formule :

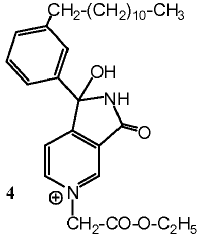

A partir du composé **2** obtenu tel que décrit dans l'Exemple 1 ci-dessus, on procède à la réaction de quaternarisation du noyau pyridine.

A une solution d'hémiamidal **2** (100 mg, 0,25 mmol) dans le tétrahydrofurane anhydre (3,2 mL) à reflux, est ajouté goutte à goutte sous atmosphère inerte l' éthyl bromo acétate (113 µL, 1,00 mmol). Après 48 h, de l'éther diéthylique (10 mL) est ajouté, le précipité formé est filtré, lavé à l'éther et séché sous vide pour donner 142 mg (78%) du sel de pyridinium **4** sous forme d'une poudre marron.

**P_{f}** : Décomposition. **IR (KBr, cm⁻¹)** : 3436, 3150, 2824, 2853, 1718, 1655, 1437, 1217. **RMN ¹H (500 MHz, DMSO-d*6*)** δ (ppm) : 10,27 (s, 1H), 9,54 (s, 1H), 9,13 (d, 1 H), 8,31 (d, 1 H), 7,75 (s, 1 H), 6,61 (s, 1 H), 7,36-7,34 (m, 2H), 7,25 (d, 1H), 5,69 (s, 2H), 3,79-3,78 (m, 2H), 2,58 (t, 2H), 1,55 (m, 2H), 1,29-1,25 (m, 21 H), 0,86 (t, 3H). **RMN ¹³C (126 MHz, DMSO-d** 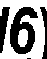 **)** δ(ppm) **:** 167,2, 166,3, 163,5, 164,5, 150,8, 143,6, 138,4, 130,2,129,6, 129,2, 126,1, 123,7, 122,3, 88,7, 60,9, 53,7, 35,7, 31,5, 31,7, 29,6-29,2, 22,6, 14,4. **SMHR (ESI)** pour C₂₉H₄₁N₂O₄ : observ. 481,3039 ; théor. 481,3066.

### EXEMPLE 4 : synthèse du composé 5 de formule :

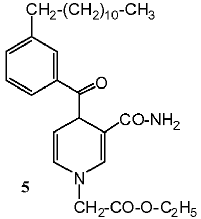

A partir du composé **4** obtenu tel que décrit ci-dessus à l'Exemple 3, on procède à la réaction de réduction du noyau pyridine.

A une solution de sel de pyridium **4** (20 mg, 0,036 mmol) dans l'éthanol (1,4 mL) est ajouté sous atmosphère inerte à 0°C, une solution de triacétoxyborohydrure de sodium (12 mg, 0,053 mmol) dans l'acide acétique (424 µL). Après 10 min sous agitation, de l'acétone (quelques gouttes) est ajouté puis de l'eau (10 mL) et le mélange réactionnel est extrait au dichlorométhane (3 x 10 mL). Les phases organiques sont réunies, lavées avec une solution saturée en bicarbonate de sodium (2 x 15 mL), séchées sur sulfate de sodium anhydre et concentrées sous pression réduite. Le résidu obtenu est purifié par chromatographie sur colonne de gel de silice (éluant : gradient dichlorométhane/ méthanol : de 100/0 à 90/10) pour donner 5,6 mg (33%) de la 1,4 dihydropyridine **5** sous forme d'une huile jaune orangée.

**RMN ¹H (300 MHz, CDCl₃)** δ (ppm) : 7,96 (m, 1 H), 7,87 (s, 1 H), 7,43-7,38 (m, 2H), 7,13 (s, 1 H), 5,89 (d, 1 H), 5,29 (s large, 2H), 5,11 (d, 1 H), 4,91 (dd, 1 H), 4,26 (q, 2H), 3,98 (s, 2H), 2,67 (t, 2H), 1,33 (t, 3H), 1,29-1,25 (m, 20H), 0,90 (t, 3H). **RMN ¹³C (126 MHz, CDCl₃)** δ(ppm) : 198,8, 169,4, 168,9, 143,5, 138,4, 135,3, 133,4, 128,5, 129,7, 129,1, 126,7, 102,8, 102,5, 61,8, 54,7, 44,1, 35,9, 31,9, 31,6, 29,7-29,3, 22,7, 14,2, 14,1. **SMHR (ESI)** pour C₂₉H₄₃N₂O₄ : observ. 483,3215 ; théor. 483,3223.

### EXEMPLES 5-7 : Synthèse du 1-(3-octyloxyphényl)-1-hydroxy-1.2-dihydro-30H-pyrrolo[3,4-c]pyridin-3-one (6), du 1-(2- dodécyloxyphényl)-1-hydroxy-1,2-dihydro-3H-pyrrolo[3,4-c]pyridin-3-one (7) et du 1-(3-octodécyloxyphényl)-1-hydroxy-1,2-dihydro-3H-pyrrolo[3,4-c]pyridin-3-one (8)

### 1^{ère} étape : Synthèse du 1-bromo -3-octyloxybenzène (6a), du 1-bromo-2-décyloxybenzène (7a) et du 1-bromo-3-octadécyloxybenzène (8a)

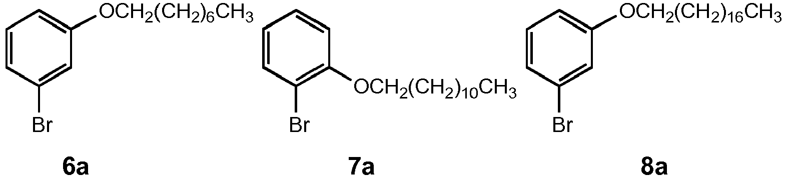

A une solution de bromophénol (1,00 g ; 5,8 mmol) dans du diméthylformamide (60 mL) est ajouté le carbonate de potassium (1,2 g ; 8,7 mmol) et le système est laissé sous agitation et sous atmosphère inerte pour 5 minutes, ensuite le 1-iodoalkane (8,67 mmol) est introduit. Après 12 h de réaction à température ambiante, 40 mL de l'eau sont ajoutés, le milieu réactionnel est extrait à l'acétate d'éthyle (3 x 30 mL), les phases organiques sont récupérées, séchées sur sulfate de sodium anhydre et concentrées sous vide. Le résidu obtenu est purifié par chromatographie sur colonne de gel de silice (éluant : cyclohexane) pour donner le composé désiré.

**Composé 6a :** 87% **IR (cm⁻¹)** : 3067, 2924 (CH), 2854, 1589 (C=C), 1466, 1243, 1227 (C-O), 1028, 860, 762, 679 (C-Br). **RMN ¹H (250MHz, CDCl₃)** δ **(ppm) :** 7,20 (m, 3H); 6,90 (dt, *J* = 1,4 Hz et 8,8 Hz, 1 H); 3,90 (t, 2H, *J* = 6,5 Hz); 1,87 (m, 2H); 1,53 (m, 10H); 0,99 (t, 3H, *J* = 4,9 Hz). **RMN ¹³C (63MHz, CDCl₃)** δ **(ppm)** : 160,0 (C); 130,5 (CH); 123,6 (CH); 122,8 (C); 117, 8 (CH); 113, 6 (CH); 68,3 (CH2); 31,9 (CH2); 29,4 (CH2); 29,3 (CH2); 26,0 (CH2); 22,7 (CH2); 14,1 (CH3). **SM (DCl/CH₄) m/z** : 285/287 (M+H⁺). **SMHR:** pour C₁₄H₂₂OBr: masse théorique : 285,0852; masse calculée: 285,0854.

**Composé 7a :** 92 %. **IR (cm⁻¹)** : 2922 (CH), 2853, 1588 (C=C), 1277, 1247 (C-O), 1051, 1030, 744, 665 (C-Br). **RMN ¹H (500MHz, CDCl₃)** δ **(ppm)** : 7,57 (dd, *J*=7,9 Hz et 1,6 Hz, 1 H); 7,28 (ddd, *J* = 1,6 Hz, 7,4 Hz et 7,4 Hz, 1 H); 6,91 (dd, *J* = 1,4 Hz et 8,2 Hz , 1 H); 6,84 (td, *J* =1,4 Hz et 7,7 Hz, 1 H); 4,05 (t, *J* = 6,6 Hz, 2H); 1,87 (dt, *J* = 6,6 Hz et 15,2 Hz, 2H); 1,60-1,30 (m, 18H); 0,89 (t, *J* = 6,2 Hz, 3H). **RMN ¹³C (126MHz, CDCl₃)** δ **(ppm)** : 155,5 (C); 133,3 (CH); 128,4 (CH); 121,6 (CH); 113,2 (CH); 112,3 (C); 69,2 (CH2); 32,0 (CH2); 29,7-22,7 (9xCH2); 14,1 (CH3). **SM (DCl/CH4) m/z :** 342,4 (M+H⁺). **SMHR:** pour C₁₈H₃₀OBr : masse théorique : 341,1464; masse calculée: 341,1480.

**Composé 8a :** 82% **IR (cm⁻¹)**: 2915 (C-H), 2847, 1597 (C=C), 1471, 1241 (C-O), 1021, 861, 782, 683 (C-Br). **RMN ¹H (250MHz, CDCl₃)** δ **(ppm):** 7,18 (m, 3H, H6) ; 6,87 (td, *J* = 0,9 Hz, 1,4 Hz et 7,9 Hz, 1 H); 3,98 (t, *J* = 6,5 Hz, 2H), 1,85 (t, *J* = 6,5 Hz, 2H); 1,49 (m, 30H); 0,93 (t, *J* = 6,1 Hz, 3H). **RMN ¹³C (63MHz, CDCl₃)** δ **(ppm)** : 160,0 (C); 130,5 (CH); 123,5 (CH); 123,0 (C); 117,8 (CH); 113,5 (CH); 68,3 (CH2); 32,0 (CH2); 29,8 - 22,7 (15 x CH2); 14,1 (CH3). **SM (DCl/CH4) m/z** : 425,2/427,2 (M+H⁺). **SMHR**: pour C₂₄H₄₂OBr : masse théorique : 425,2408; masse calculée: 425,2419.

### 2^{ème} étape : Synthèse des composés 6-8 :

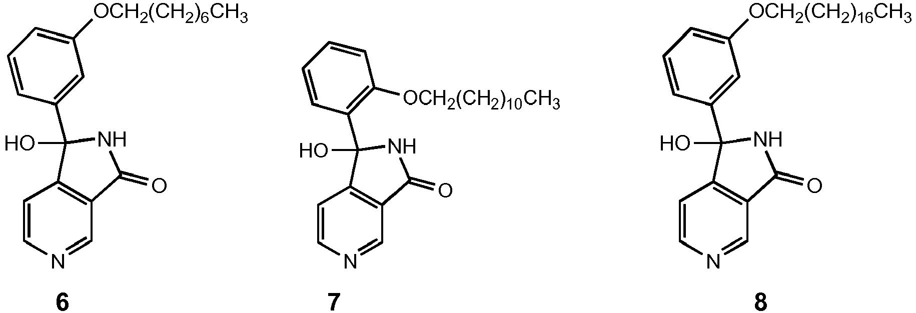

A une solution du bromoéther approprié dûmment substitué obtenu selon la 1^{ère} étape (1,21 mmoles) dans du tétrahydrofurane sec (7,5 mL), sous atmosphère inerte et à -78° C est ajouté du *tert*-butyllithium 1,5M (1,5 mL ; 2,42 mmole), au goutte à goutte. Après addition, la température est remontée à -50° C pendant 30 minutes. Puis la température de la manipulation est redescendue à -78° C et cette solution est ajoutée sur le 3,4-pyridinedicarboximide (180 mg, 1,21 mmole) dans du tétrahydrofurane anhydre (12 mL) à -78° C. Le mélange réactionnel est agité pendant une heure à -50° C. Puis, la réaction est traitée avec une solution saturée de chlorure d'ammonium (10 mL) et le milieu réactionnel est extrait à l'acétate d'éthyle (3 x 8 mL). Les phases organiques sont récupérées, séchées sur sulfate de sodium et concentrées sous vide. La poudre blanche obtenue est purifiée par chromatographie sur une colonne de gel de silice (éluant : gradientdichlorométhane/méthanol : 100/0 à 93/7) pour donner un mélange de composés *para*/*méta*. Le produit *para* **A** est récupéré proprement après 3 jours de recristallisation dans l'acétone.

**Composé 6** : 28% **IR (cm⁻¹)** : 3350 (NH, OH), 2918 (C-H), 2849, 1714 (C=O), 1613 (C=C), 1465 (C-H), 1340, 1207, 1067, 727. **RMN ¹H (500MHz, MeOD)** δ **(ppm)**: 8,93 (s, 1 H); 8,73 (d, *J* = 5,0 Hz, 1 H); 7,48 (dd, *J* = 5,1 Hz et 0,8 Hz, 2H); 7,29 (t, 1 H, *J* = 8,0 Hz); 7,18 (t, 1 H, *J* = 2,0 Hz) ; 7,07 (d, 1 H, *J* = 8,0 Hz); 6,91 (dd, *J* = 7,5 et 1,8 Hz); 4,0 (m, 2H); 1,80 (q, *J* = 6,5 Hz, 2H) ; 1,49 (m, 18H) ; 0,93 (t, *J* = 6,8 Hz, 3H). **RMN ¹³C (126MHz, MeOD)** δ **(ppm) :** 168,4 (C); 159,5 (CH); 159,4 (C); 152,5 (CH); 144,4 (CH); 141,0 (C); 129,4, 117,2, 114,3, 111,6 (4 x CH); 118,0 (C); 87,7 (C); 67,6 (CH2); 31,5 (CH2); 29,0 - 28,9 (3 x CH2); 25,7 (CH2); 22,2 (CH2); 13,0 (CH3).

**Composé 7** : 31%. **P_{f}**: = 132 °C. **IR(cm⁻¹)**: 3199 (OH, NH), 3061 (C-H), 2920, 2851, 1708 (C=O), 1619 (C=C), 1284, 1243, 1073, 1045, 1022. **RMN ¹H (500MHz, MeOD)** δ **(ppm) :** 8,90 (d, *J* =1,1 Hz, 1H); 8,69 (d, *J* = 5,3 Hz, 1H); 8,07 (dd, *J* = 1,8 Hz et 7,8 Hz, 1H); 7,37 (ddd, *J* = 1,8 Hz, 7,5 Hz et 8,1 Hz); 7,32 (dd, *J* = 1,4 Hz, et 5,1 Hz, 1H); 7,05 (dt, *J* = 1,0 Hz et 7,7 Hz, 1H); 6,88 (dd, *J* = 0,7 Hz et 8,3 Hz, 1H); 3,74 (dt, *J* = 6,3 Hz et 9,2 Hz, 1 H); 3,56 (dt, *J* = 6,3 Hz et 9,1 Hz, 1 H); 1,35 - 1,20 (m, 18H); 0,92 (t, *J* = 6,8 Hz, 3H). **RMN ¹³C (126MHz, MeOD)** δ **(ppm) :** 169,1 (C); 160,5 (C); 155,9 (C); 152,1 (CH); 143,8 (CH); 130,2 (CH); 128,5 (C); 127,8 (CH); 125,8 (CH); 119,8 (CH); 117,3 (CH); 111,6 (CH); 85,6 (C); 67,8 (CH2); 31,7 - 25,6 (9 x CH2); 22,4 (CH2); 13,1 (CH3). **SM (ESI//CH₃OH))** : 433 (M+Na⁺); 411 (M+H⁺); 393 (M+H⁺-H₂O). **SMHR**: pour C₂₅H₃₅N₂O₃: masse théorique : 411,2681; masse calculée: 411,2648.

**Composé 8** : 23% **P_{f}** : 113 °C. **IR (cm⁻¹)** : 3141 (NH, OH), 3059, 2920 (C-H), 2851, 1708 (C=O), 1607 (C=C), 1578, 1286, 1258, 1030, 700. **RMN ¹H (500MHz, MeOD)** δ **(ppm):** 8,93 (s, 1 H); 8,73 (d, *J* = 5,4 Hz, 1 H); 7,46 (d, *J* = 0,95 Hz et 5,1 Hz, 1 H); 7,29 (t, *J* = 8,0 Hz, 1 H); 7,17 (m, 1 H); 7,08 (dd, *J* = 8,8 Hz et 1,7 Hz, 1 H); 6,91 (dt, *J* = 8,3 Hz, autre *J* non-mesurable, 1 H); 3,99 (m, 2H); 1,78 (q, *J* = 6,7 Hz, 2H); 1,48-1,31 (m, 30H); 0,92 (t, *J* = 6,8 Hz, 3H). **RMN ¹³C (125MHz, MeOD)** δ **(ppm) :** 1 carbone quaternaire absent, 168,5 (C); 159,5 (C); 159,4 (C); 152,6 (CH); 144,5 (C); 141,1 (C); 129,4 (CH), 126,4 (C); 117,9 (CH), 117,4 (C), 114,5 (CH); 111,8 (CH); 67,9 (C); 31,6 (CH2); 29,3 - 28,9 (13 x CH2); 25,7 (CH2) ; 22,2 (CH2); 12,9 (CH3). **SM (ESI//CH₃OH))** : 495,5 (M+H⁺). **SMHR**: pour C₃₁H₄₇N₂O₃: masse théorique : 495,3618; masse calculée: 495,3587.

### EXEMPLE 8 : Synthèse du 1-[3-(dodécylthio)phényl]-1-hydroxy-1,2-dihydro-3H-pyrrolo[3,4-c]pyridin-3-one (9)

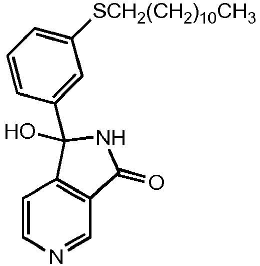

### 1^{ère} étape : Synthèse du 1-bromo-3-(dodécylthio)benzène (9a)

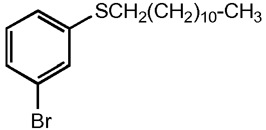

A une solution de 3-bromobenzène thiol (1,50 g ; 7,9 mmol) dans du diméthylformamide (90 mL) est ajouté le carbonate de potassium (1,6 g ; 11,8 mmol) et le système est laissé sous agitation et sous atmosphère inerte pour 5 minutes, ensuite le 1-iodododécane (3,5 g ; 11,8 mmol) est introduit. Après 16 h de réaction à température ambiante, 50 mL de l'eau sont ajoutés, le milieu réactionnel est extrait à l'acétate d'éthyle (3 x 30 mL), les phases organiques sont récupérées, séchées sur sulfate de sodium anhydre et concentrées sous vide. Le produit obtenu **9a**, sous forme d'huile incolore (4,5 g) est purifié par chromatographie sur colonne de gel de silice (éluant : cyclohéxane) pour obtenir 1,9g (70%).

**IR (cm⁻¹)** : 2921 (C-H), 2851 (C-H), 1576 (C=C), 1458 (C-S), 1068, 753, 676 (C-Br); **RMN ¹H (250MHz, CDCl₃)** δ **(ppm):** 7,46 (dd, 1 H, *J* = 6,7 Hz et 1,6 Hz); 7,23-7,32 (m, 2H, H4); 7,16 (dd, 1 H, *J* = 12,0 Hz et 7,8 Hz); 2,97 (t, 2H, *J* = 7,2 Hz); 1,65 (t, 2H, *J* = 7,3 Hz); 1,29 (m, 18H); 0,91 (t, *J* = 6,6 Hz, 3H). **RMN ¹³C (63MHz, CDCl₃)** δ **(ppm) :** 139,8 (C); 130,7 (CH); 130,0 (CH); 128,5 (CH); 126,9 (C); 122,8 (C); 33,3 (CH2); 31,9 (CH2); 29,7-28,8 (8 x CH2); 22,7 (CH2) ; 14,1 (CH3). **SM (DCl/CH₄) m/z:** 357 (M+H⁺); 385 (M + C₂H₅⁺). **SMHR:** pour C₁₈H₃₀SBr: masse théorique : 357,1236; masse calculée: 357,1252.

### 2^{ème} étape : Synthèse du composé (9) :

A une solution de bromobenzène **9a** (1,00 g ; 2,8 mmole) dans du tétrahydrofurane sec (18 mL), sous atmosphère inerte et à -78° C est ajouté du *n-*butyllithium 1,5M (1,4 mL ; 3,36 mmole), au goutte à goutte. Après addition, la température est remontée à -50° C pendant 30 minutes. Puis la température de la manipulation est redescendue à -78° C et cette solution est ajoutée sur le 3,4-pyridinedicarboximide (166 mg, 1,12 mmole) dans du tétrahydrofurane anhydre (22 mL). Le mélange réactionnel est agité pendant une heure à -50 ° C. Puis, la réaction est traitée avec une solution saturée de chlorure d'ammonium (30 mL) et le milieu réactionnel est extrait à l'acétate d'éthyle (3 x 20 mL). Les phases organiques sont récupérées, séchées sur sulfate de sodium et concentrées sous vide. La poudre blanche obtenue (720 mg) est purifiée par chromatographie sur une colonne de gel de silice (éluant : gradient dichlorométhane/méthanol : 100/0 à 93/7) pour obtenir 128 mg (48 %) du mélange de composés *para*/*méta.* Le produit *para* est récupéré (32,3 mg ; 28 %) après 3 jours de recristallisation dans l'acétone. Ce produit de recristallisation est encore contaminé par 23% du composé *méta*.
**P_{f}**: = 86 °C. **IR (cm⁻¹)**: 3159 (NH, OH), 2917 (C-H), 2848 (C-H), 2424, 1698 (C=O), 1614 (C=C), 1547,1464 (C-S), 1347, 1067, 964 (C=C), 784, 698. **RMN ¹H (250MHz, MeOD)** δ **(ppm) :** 8,94 (s, 1H); 8,74 (d, 1 H, *J* = 5,0 Hz); 7,56 (s, 1H); 7,46 (d, 1 H, *J* = 4,6 Hz, 1 H); 7,31 (m, 3H, H14); 2,96 (t, 2H, *J* = 7,0 Hz); 1,64 (m, 2H); 1,42 (m, 18H); 0,94 (t, 3H, *J* = 6,0 Hz). **RMN ¹³C (126 MHz, MeOD)** δ **(ppm) :** 159,0 (C); 152,7 (CH); 150,0 (C); 144,4 (CH); 144,0 (C); 140,0 (C); 138,0 (C); 128,9 (CH), 128,4 (CH); 125,2 (CH), 122,5 (CH); 118,0 (CH, C5); 88,0 (C), 71,0 (CH2); 32,6 (CH2); 31,7 (CH2); 29,4-28,4 (CH2) ; 22,3 (CH2); 13,0 (CH3, C29). **SM (ESI//CH₃OH)):** 449,2 (M+Na⁺); 427,3 (M+H⁺). **SMHR:** pour C₂₅H₃₅N₂O₂S: masse théorique : 411,2681; masse calculée: 411,2648.

### EXEMPLE 9 : Synthèse du 1-(3-dodécyloxyphényl)-1-méthoxy-1,2-dihydro-3H-pyrrolo[3,4-c]pyridin-3-one (10)

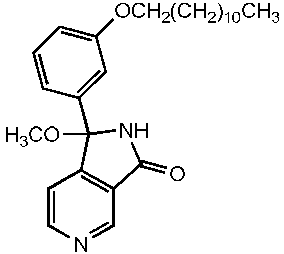

A une solution de **3** (100 mg ; 0,44 mmol) dans du méthanol séché sur tamis moléculaire (6 mL) sont ajoutés 0,5 mL de chlorure de thionyle. Le tout est porté à reflux sous atmosphère d'argon pour 16 heures. Le chauffage est arrêté et lorsque le milieu atteint la température ambiante, il est traité avec une solution saturée de bicarbonate de sodium (5 mL) et extrait à l'acétate d'éthyle (3 x 8 mL). Les phases organiques sont récupérées, séchées sur sulfate de sodium et concentrées sous vide pour donner 93 mg (89%) d'huile jaune.
**IR (cm⁻¹)** : 2922 (C-H), 2852, 1726 (C=O), 1603 (C=C), 1438, 1287 (C-O), 1051, 698.
**RMN ¹H (250MHz, CDCl₃)** δ **(ppm)**: 9,08 (s, 1 H), 8,78 (s, 1 H), 7,39-6,89 (m, 6H), 3,97 (t, *J* = 6,4 Hz, 2H), 3,17 (s, 3H), 1,79 (t, *J* = 5,5 Hz, 2H), 1,45-1,28 (m, 18H), 0,91 (t, *J* = 5,9 Hz, 3H). **RMN ¹³C (63MHz, CDCl₃)** δ **(ppm)**: 168,1 (C); 159,6 (CH); 154,9 (C); 153,2 (CH); 146,0 (CH); 139,7 (2 x C); 130,0 (CH), 118,0 (C) 117,4 (CH), 114,8 (CH) ; 112,1 (CH); 91,9 (C); 68,2 (CH2); 50,9 (CH3); 31,9 (CH2); 29,6 - 29,3 (6 x CH2); 26,0 (CH2); 22,7 (CH2), 14,1 (CH2); 1,0 (CH3). **SM (ESI/CH₃OH)) m/z:** 425,6 (M+H⁺). **SMHR:** pour C₂₆H₃₇N₂O₃: masse théorique : 425,2847; masse calculée: 425,2804.

Les composés suivants ont été synthétisés par application ou adaptation des méthodes décrites ci-avant au moyen des produits de départ et réactifs appropriés :

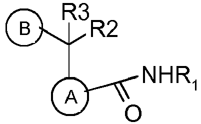

| Ex. | Ⓐ | Ⓑ | R1 | R2 | R3 |
|---|---|---|---|---|---|
| 1 | 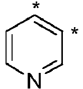 | 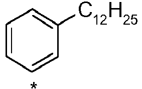 | forment ensemble une liaison simple | | OH |
| 2 | 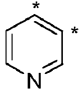 | 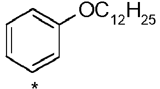 | forment ensemble une liaison simple | | OH |
| 3 | 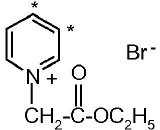 | 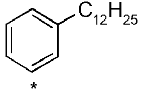 | forment ensemble une liaison simple | | OH |
| 4 | 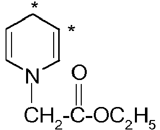 | 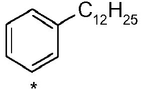 | H | forment ensemble un groupe * =O | |
| 5 | 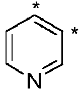 | 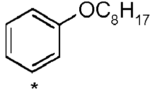 | forment ensemble une liaison simple | | OH |
| 6 | 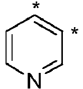 | 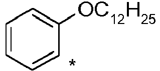 | forment ensemble une liaison simple | | OH |
| 7 | 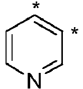 | 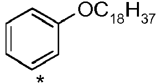 | forment ensemble une liaison simple | | OH |
| 8 | 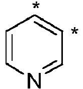 | 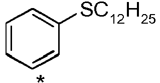 | forment ensemble une liaison simple | | OH |
| 9 | 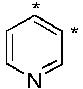 | 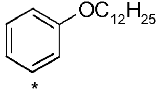 | forment ensemble une liaison simple | | OCH₃ |
| 10 | 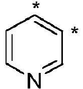 | 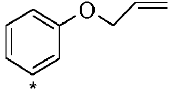 | forment ensemble une liaison simple | | OH |
| 11 | 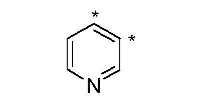 | 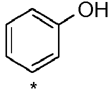 | forment ensemble une liaison simple | | OH |
| 12 | 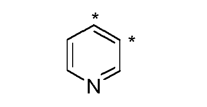 | 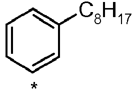 | forment ensemble une liaison simple | | OH |
| 13 | 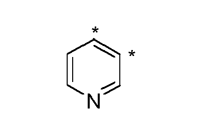 | 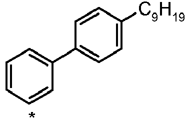 | forment ensemble une liaison simple | | OH |
| 14 | 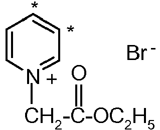 | 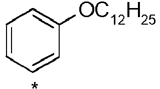 | forment ensemble une liaison simple | | OH |
| 15 | 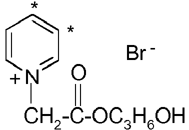 | 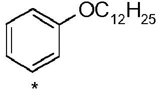 | forment ensemble une liaison simple | | OH |
| 16 | 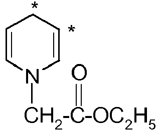 | 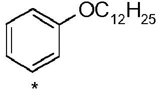 | H | forment ensemble un groupe * =O | |
| 17 | 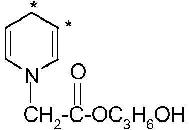 | 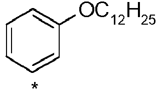 | H | forment ensemble un groupe * =O | |

Dans le tableau ci-dessus, les astérisques (*) désignent les positions d'attache et de substitution des noyaux A et B.

### EXEMPLE 18 : mise en évidence de l'effet inhibiteur des composes revendiques sur l'activité de InhA.

### 1 - Préparation de l'énoylréductase InhA.

La protéine InhA a été exprimée dans *E*. *coli* après clonage du gène *inhA* dans un vecteur plasmidique de type pET. La croissance de la souche résultante et l'induction de l'expression du gène *inhA* par 1 mM d'IPTG (isopropyl β-D-thiogalactoside) conduit à la production de la protéine InhA hydrosoluble d'environ 28,5 KDa (28 368 Da) (monomère) qui est purifiée par des techniques standards de purification de protéines. L'enzyme existe à l'état de tétramère en solution et est conservée dans du tampon Hepes 50 mM, 50% glycérol à -20°C.

### 2 - Evaluation de l'activité de l'énovlréductase InhA.

L'activité énoyl réductase d'InhA est suivie par spectrométrie UV en suivant la disparition du signal du cofacteur réduit NADH à 340 nm en fonction du temps. Le pourcentage d'inhibition est calculé en soustrayant à 100 le rapport des vitesses initiales (V) mesurées lors de cinétiques avec et sans inhibiteur et multiplié par 100 : % inhibition = 100 - (V_{inhib}/V_{blanc}*100). La vitesse initiale est mesurée en traçant la tangente de la courbe DO = f(temps) au temps zéro.

### 3 - Test d'inhibition d'activité de l'énoylréductase InhA.

La réaction enzymatique est effectuée dans un volume final de 100 µL (en cuve de quartz, trajet optique 1 cm). L'absorption de chaque mélange réactionnel est déterminée avec un spectrophotomètre UVIKON 293 (Bio-Tek Kontron Instruments) relié à un bain thermostaté permettant de réguler la température de la cuve à 25°C. Une ligne de base est effectuée pendant la préincubation juste avant le début des mesures. Les mesures sont effectuées sur 3 min après une préincubation de 5 min ou de 2 h.

La préincubation est réalisée dans 90 µL (volume total) d'une solution de tampon PIPES 30 mM, NaCl 150 mM, pH = 6,8 à 25°C contenant 100 nM d'InhA, 100 µM, 20 µM ou 10 µM du composé à tester (ou 500 nM du pool d'adduits INH-NAD constituant des inhibiteurs-contrôle) et 200 µM de NADH. Après 5 min ou 2 h de préincubation, l'addition de 35 µM du substrat 2-*trans*-décénoyl-CoA initie la réaction. Ou bien la préincubation est réalisée dans 80 µL (volume total) d'une solution de tampon de PIPES 30 mM, NaCl 150 mM, pH = 6,8 à 25°C contenant 100 nM d'InhA et 100 µM, 20 µM ou 10 µM du composé à tester (ou 500 nM du pool d'adduits constituant des inhibiteurs contrôle). Après 5 min ou 2 h de préincubation, l'addition de 200 µM de NADH et de 35 µM du 2-*trans*-décénoyl-CoA initie la réaction.

### 4 - Résultats

Les résultats obtenus montrent que certains dérivés de la famille moléculaire faisant l'objet de l'invention présentent une activité inhibitrice efficace du fonctionnement d'InhA, proche de celle des métabolites actifs de l'isoniazide (obtenus par synthèse biomimétique sous forme d'un mélange d'adduits). Les pourcentages d'inhibition d'InhA en fonction de la concentration en inhibiteur et pour 5 min (ou * 2h) de temps de préincubation sont illustrés ci-dessous :

| | | |
|---|---|---|
| Mélange d'adduits INH-NAD de référence : | 10 µM | 92 % |
| Composé de l'exemple 2 | 100 µM | 41 % (* 80 %) |
| Composé de l'exemple 3 | 100 µM | 91 % |
| | 10 µM | 19 % |
| Composé de l'exemple 4 | 10 µM | 25 % |

### EXEMPLE 19 : mise en évidence de l'inhibition de croissance des composés selon l'invention sur différentes souches bactériennes : cas de mycobacterium smegmatis.

### 1 - Préparation de la suspension bactérienne de Mycobacterium smegmatis.

La souche *Mycobacterium smegmatis* mc²155 (R₀) est cultivée dans un milieu de culture Middelbrook 7H9(Difco) + glycérol 0,2% + 0,05% tween 80 à 37°C sous agitation (200 rpm) afin d'éviter la formation de voile bactérien. Après 3 jours, la culture R₀ est laissée au repos 10 min, le temps que les plus gros agrégats sédimentent. Le surnageant est prélevé pour lancer une nouvelle culture R₁, dans les mêmes conditions, ensemencée au 1/100. Après 3 jours sous agitation à 37°C, et 10 min de repos, le surnageant est prélevé. La densité optique de la suspension bactérienne est mesurée à 650 nm et ramenée à une valeur comprise entre 0,002 et 0,003 par dilution dans le milieu de culture (sans tween 80), c'est la R₂.

### 2 - Evaluation de la croissance de la suspension bactérienne de Mycobacterium smegmatis.

L'activité antimycobactérienne est évaluée par des tests colorimétriques de réduction du MTT (bromure de 3-(4,5-diméthylthiazol-2-yl)-2,5-diphényl-2H-tétrazolium), sur la souche *M. smegmatis* mc²155. Lorsque que les bactéries ont pu se développer normalement, le sel de tétrazolium (MTT) jaune se réduit et change de couleur, il devient violet. Par contre si la croissance des bactéries est complètement inhibée par un de nos composés, la réduction ne peut plus avoir lieu et la solution reste jaune. Une lecture de la densité optique à 570 nm permet d'observer la formation du MTT formazan réduit de couleur violette. Le pourcentage d'inhibition est calculé en soustrayant à 100 le rapport des DO sans et avec inhibiteur multiplié par 100 : 100 - (DOᵢₙₕ./DOₜₑₘ * 100)

### 3 - Activité inhibitrice des produits à tester sur la croissance de la suspension bactérienne de Mycobacterium smegmatis.

Les essais sont réalisés sur des microplaques NUNC (Merk-eurolab) 96 puits. Chaque produit est testé sur une gamme de concentration à partir de 5 mM en réalisant des dilutions successives de deux en deux. Chaque puits contient 100 µL de composé en solution dans le milieu de culture (7H9 + 0,2% Gro avec 1% (v/v) de DMSO final). Il est ensuite ajouté 100 µL de suspension bactérienne R₂. La plaque est fermée par du parafilm et incubée à 37°C. Après 24 heures d'incubation, 50 µL d'une solution à 1 mg/mL de MTT sont rajoutés dans chaque puits. Après 3 heures d'incubation à 37°C, 100 µL de tampon de lyse sont ajoutés dans chaque puits et la plaque est laissée sous agitation à température ambiante jusqu'à ce que la solution soit bien homogène.

La densité optique est mesurée à 570 nm avec un spectrophotomètre lecteur de microplaques µQuant Bio-tek instruments, INC.

### 4 - Résultats

Les résultats obtenus montrent que certains dérivés de la famille faisant l'objet de l'invention présentent des valeurs de CI₅₀ (concentration correspondant à 50 % d'inhibition) sur la croissance de la souche *Mycobacterium smegmatis* meilleures que celles observées pour l'isoniazide pris en référence.

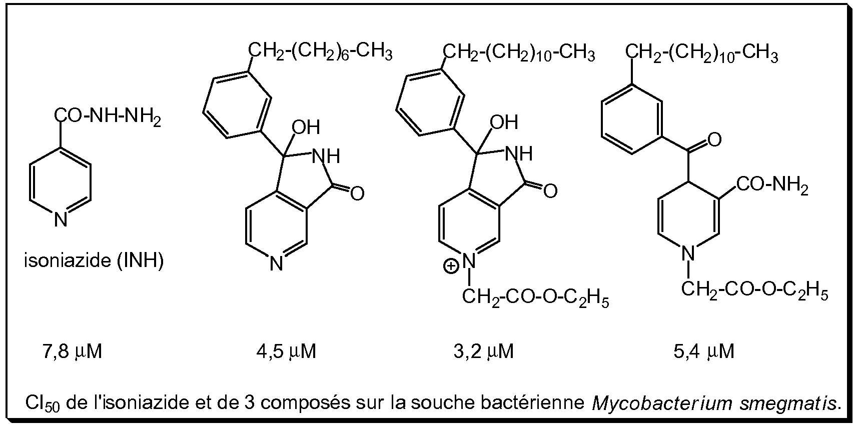

### EXEMPLE 19 : mise en évidence de l'inhibition de croissance des composés selon l'invention sur différentes souches bactériennes : cas de Corynebacterium glutamicum

La CI₅₀ de composés selon l'invention sur la souche bactérienne C. *glutamicum* ainsi que pour le témoin INH a été déterminée.

Comme attendu, INH ne présente aucune activité sur C. glutamicum à 5mM.

Les composés selon l'invention, notamment décrits ci-après sont capables d'inhiber la croissance de la bactérie C. *glutamicum* à une CI₅₀ inférieure ou égale à 10 µM. Ces résultats suggèrent que les composés cités ci-dessous présentent en plus d'InhA, une autre cible moléculaire.

En effet, les bactéries appartenant à cette souche présentent la particularité de ne pas posséder de cycle d'élongation FAS-II dans la biosynthèse des acides gras. Il n'y a pas d'équivalent d'InhA dans C. *glutamicum.* Les composés selon l'invention actifs sur cette souche de bactéries ont donc également une autre cible que le système FAS-II

### Préparation de la suspension bactérienne de C. glutamicum.

La souche de *Corynebacterium glutamicum* est cultivée dans un milieu BHI (Brai Heart Infusion) à 30°C sous agitation à 200 rpm. La DO de la culture est directement mesurée et ajustée à une valeur de 0,002-0,003 par dilution dans le milieu de culture LB. *C*. *glutamicum* pousse aussi dans le milieu LB, on l'utilise à la place du BHI car ce dernier est très coloré et perturbe la lecture de la DO à 570 nm.

### Préparation des solutions de composés à tester.

Les gammes de concentration testées sur *C*. *glutamicum* sont décrites dans le tableau.

| *Composés* | *Gamme de concentration testée* |
|---|---|
| INH | 5 mM à 2.4 µM |
| A | 156 µM à 2.4 µM |
| Exemple 2 | 312 µM à 4,9 µM |
| Exemple 4 | 78 µM à 1,2 µM |
| Exemple 5 | 39 µM à 0,6 µM |

Les solutions-mères sont préparées dans le DMSO à une concentration 100 fois supérieure à la plus forte concentration à tester.

### Evaluation de la croissance de la suspension bactérienne de C. glutamicum

Le test se déroule de la même façon que pour le test sur *M. smegmatis.*

Les essais sont réalisés sur des microplaques NUNC (Merck-eurolab) 96 puits. Toutes les concentrations ont été testées au moins deux fois.

Chaque puits contient 100 µL de composé en solution dans le milieu de culture (LB avec 1% de DMSO final). Il est ensuite ajouté 100 µl de suspension bactérienne de 0.002 de DO à 650 nm.

La plaque est fermée par du parafilm et incubée à 30°C pour *C. glutamicum.* Après deux heures d'incubation, 50 µL d'une solution à 1 mg/mL de sel de tétrazolium (MTT) sont rajoutés dans chaque puits. Après une heure d'incubation à 30°C pour C. *glutamicum,* 100 µL de tampon de lyse sont ajoutés dans chaque puits et la plaque est laissée sous agitation à température ambiante jusqu'à ce que la solution soit bien homogène.

La densité optique est mesurée à 570 nm avec un spectrophotomètre µQuant Bio-tek instruments, INC, lecteur de plaque 96 puits.

### Résultats

Les CI₅₀ de l'isoniazide et de 4 composés sur la souche bactérienne *Corynebacterium glutamicum* sont illustrés ci-après :

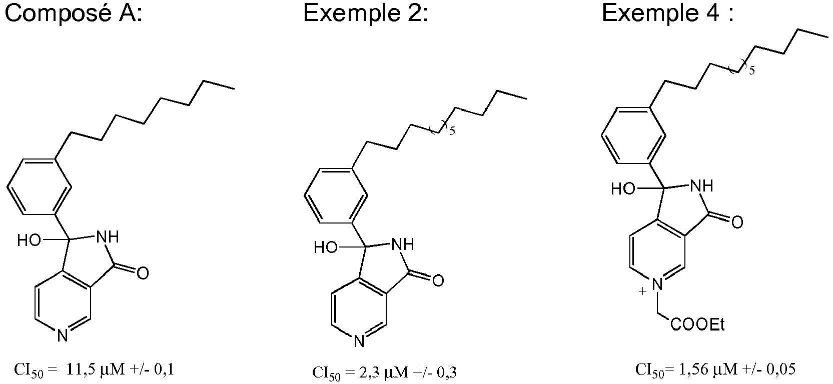

### Exemple 5 : Isoniazide CI₅₀ > 5 mM

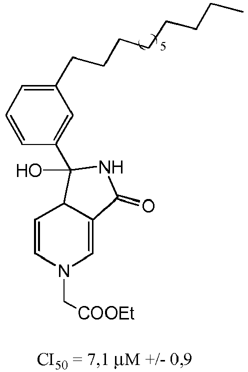

Les résultats obtenus montrent la capacité de composés selon l'invention d'inhiber la croissance de *C*. *glutamicum* (souche bactérienne sans équivalent d'InhA), ce qui laisse penser que d'autres cibles peuvent exister.

## Revendications

1. Composés de formule générale (I) : 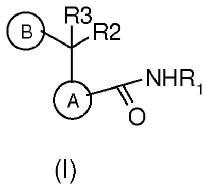 dans laquelle :
- le cycle Ⓐ représente un noyau aromatique ou non aromatique, de 6 chaînons, comportant éventuellement un ou plusieurs atomes d'azote, le(s) dit(s) atome(s) d'azote pouvant être éventuellement substitué(s) par
un groupe tétrahydrofurane éventuellement substitué, ou par
un groupe -CH₂-E où E est choisi parmi -CONRR' ; -CO-SR ; -CO-OAlkyle ; -CO-OAlkyle-OH ; -O-Alkyle-OAc ; phényle substitué par exemple par un groupe CN ou NO₂ ; où R et R', identiques ou différents représentent indépendamment un atome d'hydrogène ou un groupe alkyle ;
et/ou le(s)dit(s) atome(s) d'azote pouvant être présent(s) sous forme de sels de pyridinium, le contre-ion étant alors l'anion d'un atome d'halogène,
- Ⓑ représente un groupe aryle ou hétéroaryle, mono ou bicyclique, de 5 à 10 chaînons, substitué par un ou plusieurs groupes choisis parmi les groupes - -(C₅-C₂₀)Alkyle ; -O(C₂-C₂₀)Alkyle ; -S(O)ₚAlkyle où p=0, 1 ou 2 ; Alkényle; Alkynyle ;
- R1 représente un atome d'hydrogène ou un groupe Alkyle et R2 et R3 forment ensemble un groupe =O ;
ou, alternativement,
R3 représente un groupe -OH ou -OAlkyle et R2 forme avec R1 une liaison simple liant l'atome d'azote à l'atome de carbone substitué par R3, Ⓐ et Ⓑ de façon à former un noyau indanone par fusion avec le cycle Ⓐ ;
Etant entendu que les groupes -C(=O)NHR1 et C(R3)(R2)- sont situés sur deux positions adjacentes du noyau Ⓐ ;
à l'état de base ou de sel d'addition à un acide, ainsi qu'à l'état d'hydrate ou de solvat pharmaceutiquement acceptables.

2. Composés selon la revendication 1 tels que dans la formule générale (I) Ⓐ est un noyau phényle, pyridine, pyrazine ou dihydropyridine.

3. Composés selon la revendication 1 ou 2 tels que dans la formule générale (I) Ⓐ représente un groupe dihydropyridine ou pyridine, éventuellement substitué par un groupe choisi parmi les groupes -CH₂-CO-OAlkyle, -CH₂-CO-OAlkyle-OH, -CH₂-O-Alkyle-OAc, et/ou éventuellement sous forme d'halogénure de pyridinium.

4. Composés selon l'une quelconque des revendications précédentes, tels que dans la formule générale (I), Ⓑ est un noyau phényle substitué par un groupe (C₅-C₂₀)Alkyle, O(C₅-C₂₀)Alkyle, -S(O)ₚAlkyle où p=0, 1 ou 2.

5. Composés selon l'une quelconque des revendications précédentes tels que dans la formule générale (I) Ⓑ est substitué en position ortho ou méta.

6. Composés selon l'une quelconque des revendications précédentes tels que dans la formule générale (I) R3=OH ou -OAlkyle et R1 et R2 forment ensemble une simple liaison.

7. Composés selon l'une quelconque des revendications 1 à 5 tels que dans la formule générale (I) R3 et R2 forment un groupe =O et R1 représente un atome d'hydrogène.

8. Composés selon l'une quelconque des revendications précédentes tels que dans la formule générale (I) :
Ⓐ représente un groupe dihydropyridine, éventuellement substitué par un groupe choisi parmi -CH₂COOAlkyle, -CH₂COOAlkyleOH ;
Ⓑ représente un groupe phényle, substitué en position méta par un groupe choisi parmi les groupes C₅-C₂₀ alkyle, -O(C₂-C₂₀)Alkyle ;
R1 et R2 forment ensemble une liaison simple et R3=OH, ou R1=H et R2 et R3 forment ensemble un groupe =O,
à l'état de base ou de sel d'addition à un acide, ainsi qu'à l'état d'hydrate ou de solvat pharmaceutiquement acceptables.

9. Composés selon la revendication 1, tels que le groupe tétrahydrofurane éventuellement substitué est le 2-hydroxyméthyl-tétrahydrofurane-3,4-diol.

10. Composés selon la revendication 1, tels que le cation du pyridinium est le bromure.

11. Composés selon l'une quelconque des revendications précédentes choisis parmi les composés suivants :
- 1-(3-dodécylphényl)-1-hydroxy-1,2-dihydropyrrolo[3,4-c]pyridin-3-one ;
- 1-(3-dodécyloxyphényl)-1-hydroxy-1,2-dihydropyrrolo[3,4-c]pyridin-3-one ;
- bromure de 1-(3-dodécylphényl)-5-[2-(éthyloxy)-2-oxoéthyl]-1-hydroxy-3-oxo-1,2-dihydropyrrolo[3,4-c]pyridinium;
- 3-aminocarbonyl-[4-(3-dodécylbenzoyl)-1,4-dihydro-pyridin-1-yl] acétate d'éthyle ;
- 1-(3-octyloxyphényl)-1-hydroxy-1,2-dihydro-3*H*-pyrrolo[3,4-*c*]pyridin-3-one ;
- 1-(2-dodécyloxyphényl)-1-hydroxy-1,2-dihydro-3*H*-pyrrolo[3,4-*c*]pyridin-3-one ;
- 1-(3-octodécyloxyphényl)-1-hydroxy-1,2-dihydro-3*H*-pyrrolo[3,4-*c*]pyridin-3-one ;
- 1-[3-(dodécylthio)phényl]-1-hydroxy-1,2-dihydro-3*H*-pyrrolo[3,4-*c*]pyridin-3-one ;
- 1-(3-dodécyloxyphényl)-1-méthoxy-1,2-dihydro-3*H*-pyrrolo[3,4-*c*]pyridin-3-one ;
- 1-Hydroxy-1-(3-(propèn-3-yl)oxyphényl)-1,2-dihydropyrrolo[3,4-c]pyridin-3-one ;
- 1-Hydroxy-1-(3-octylphényl)-1,2-dihydropyrrolo[3,4-*c*]pyridin-3-one ;
- bromure de 1-[3-(dodécyloxy)phényl]-5-(2-éthoxy-2-oxoéthyl)-1hydroxy-3-oxo-2,3-dihydro-1*H*-pyrrolo[3,4-*c*]pyridin-5-ium ;
- bromure de 1-[(3-(dodécyloxy)phényl)-1-hydroxy-5-[2-(3-hydroxypropoxy)-2-oxyéthyl]-3-oxo-2,3-dihydro-1*H*-pyrrolo[3,4-c]pyridin-5-ium ;
- 3-aminocarbonyl-[4-(3-dodécyloxybenzoyl)-1,4-dihydropyridin-1-yl]acétate d'éthyle ;
- 3-aminocarbonyl-[4-(3-dodécyloxybenzoyl)-1,4-dihydropyridin-1-yl]acétate de 3-hydroxypropyle ;
à l'état de base ou de sel d'addition à un acide ainsi qu'à l'état d'hydrate ou de solvat pharmaceutiquement acceptables.

12. Procédé de préparation d'un composé de formule générale (I) selon l'une quelconque des revendications précédentes dans laquelle R1 et R2 forment ensemble une liaison simple et R3 représente -OH comprenant l'étape de couplage d'un composé de formule générale (II) : 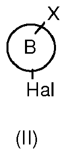 et d'un composé de formule générale (III) : 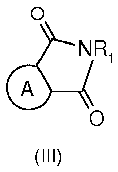 conduisant au composé de formule (IV) : 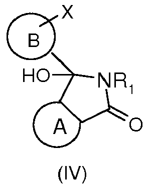 dans lesquelles Ⓐ, Ⓑ et R1 sont définis tel qu'en formule générale (I), Hal représente un atome d'halogène et X représente soit un atome d'hydrogène lorsque Ⓑ n'est pas substitué, soit le substituant de Ⓑ correspondant à la formule générale (I) désirée (dans ces deux cas, le composé (IV) correspond au composé (I)), soit un atome d'halogène le couplage étant suivi dans ce cas de la réaction de substitution de l'atome d'halogène du composé (IV) par le substituant de Ⓑ approprié correspondant à la formule générale (I) désirée.

13. Procédé de préparation d'un composé de formule générale (I) selon l'une quelconque des revendications 1 à 9 dans laquelle R1 et R2 forment ensemble une liaison simple et R3 représente -OH comprenant l'étape de cyclisation de composés de formule générale (V) 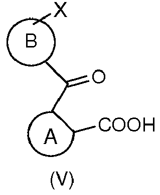 dans laquelle Ⓐ, Ⓑ et Hal sont définis comme en formule générale (IV) en présence de NHR1 (V'),
conduisant au compose de formule (IV) : 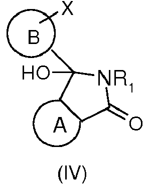 dans lesquelles R1 est défins tel qu'en formule générale (I), et X représente soit un atome d'hydrogène lorsque Ⓑ n'est pas substitué, soit le substituant de Ⓑ correspondant à la formule générale (I) désirée (dans ces deux cas, le composé (IV) correspond au composé (I)), soit un atome d'halogène la cyclisation étant alors suivie dans ce cas de la réaction de substitution de l'atome d'halogène du composé (IV) par le substituant de Ⓑ approprié correspondant à la formule générale (I) désirée.

14. Procédé, selon la revendication 12 ou 13, de préparation d'un composé de formule générale (I) dans laquelle Ⓐ représente un noyau pyridinium dans lequel l'atome d'azote est quaternarisé, le procédé de préparation comprenant en outre l'étape ultérieure consistant à quaternariser le composé de formule générale (I) dans lequel Ⓐ représente un noyau pyridine au moyen d'un composé de formule générale (XII) :
R-Hal (XII)
dans laquelle Hal représente un atome d'halogène et R représente un groupe de type -CH₂-E ou E est un groupe électroattracteur tel que défini en revendication 1.

15. Procédé de préparation d'un composé de formule générale (I) selon l'une quelconque des revendications 1 à 11, dans laquelle Ⓐ représente un noyau dihydropyridine et R2 et R3 forment ensemble un groupe =O et R1 représente un atome d'hydrogène, ledit procédé comprenant le procédé selon la revendication 12 suivi de l'étape de réduction du composé de formule générale (I) dans laquelle Ⓐ représente un noyau pyridinium.

16. Procédé selon l'une quelconque des revendications 12 à 15 comprenant en outre la réaction d'isolement et/ou de purification du produit obtenu.

17. Composition pharmaceutique comprenant, à titre de principe actif, un composé de formule générale (I) tel que défini selon l'une quelconque des revendications 1 à 11
et au moins un véhicule pharmaceutiquement acceptable.

18. Combinaison d'un composé de formule générale (I) selon l'une quelconque des revendications 1 à 11 et un principe actif à activité anti-infectieuse.

19. Utilisation d'un composé selon l'une quelconque des revendications 1 à 11 pour la préparation d'un médicament anti-infectieux.

20. Composé de formule générale selon l'une quelconque des revendications 1 à 11 pour son utilisation pour le traitement et/ou la prévention des infections.

21. Composé pour utilisation selon la revendication 20 tel que lesdites infections humaines ou animales sont choisies parmi les mycobactérioses, la lèpre ou les maladies opportunistes comme celles liées à *Mycobacterium avium*, *M. bovis* et/ou *M. ulcerans, M. marinum* ; le paludisme ou toute infection liée à un agent pathogène possédant une enzyme de type enoyl-Acyl Carrier Protein reductase ou une enzyme de structure apparentée, appartenant à la superfamille structurale des Short chain Dehydrogenases Reductases (SDR).

22. Composé pour utilisation selon la revendication 21, telle que les mycobactérioses sont choisies parmi la tuberculose, la lèpre ou les maladies opportunistes comme celles liées à *Mycobacterium avium, M. bovis* et/ou *M*. *ulcerans, M. marinum*.

23. Composé pour son utilisation selon la revendication 21 ou 22 pour son utilisation pour le traitement et/ou la prévention de la tuberculose.

## Patentansprüche

1. Verbindungen der allgemeinen Formel (I) : 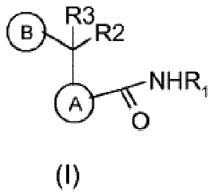 wobei:
- der Ring Ⓐ einen aromatischen oder nicht-aromatischen 6-gliedrigen Ring darstellt, welcher gegebenenfalls ein oder mehrere Stickstoffatom(e) aufweist, wobei das/die Stickstoffatom(e) gegebenenfalls substituiert werden kann/können mit
einer gegebenenfalls substituierten Tetrahydrofuran-Gruppe, oder mit einer -CH₂-E-Gruppe, wobei E ausgewählt ist aus -CONRR'; -CO-SR; -CO-OAlkyl; -CO-OAlkyl-OH; -O-Alkyl-OAc; Phenyl substituiert beispielsweise mit einer CN- oder NO₂-Gruppe; wobei R und R' identisch oder verschieden unabhängig ein Wasserstoffatom oder eine AlkylGruppe darstellen;
und/oder das/die Stickstoffatom(e) in Form eines Pyridinium-Salzes anwesend sein kann/können, wobei das Gegen-Ion dann das Anion eines Wasserstoffatoms ist,
- Ⓑ eine Aryl- oder Heteroaryl-, mono oder bicyclische, 5 bis 10-gliedrige Gruppe darstellt, substituiert mit einer oder mehreren Gruppen ausgewählt aus den Gruppen -(C₅-C₂₀)Alkyl; -O(C₂-C₂₀)Alkyl; - S(O)ₚAlkyl wobei p = 0, 1 oder 2; Alkenyl; Alkynyl;
- R1 ein Wasserstoffatom oder eine Alkyl-Gruppe darstellt und R2 und R3 zusammen eine =O-Gruppe bilden;
oder, alternativ
R3 eine OH- oder OAlkyl-Gruppe darstellt und R2 mit R1 eine Einfachbindung bilden, welche das Stickstoffatom mit dem mit R3, Ⓐ und
Ⓑ substituierten Kohlenstoffatom bindet, um einen Indanonring durch Schmelzen mit dem Ring Ⓐ zu bilden;
wobei die -C(=O)NHR1 und C(R3)(R2)- Gruppen sich an zwei benachbarten Positionen des Rings Ⓐ befinden;
im Zustand einer Base oder eines Säureadditionssalzes, sowie im Zustand eines Hydrates oder eines Solvates, welche pharmazeutisch verträglich sind.

2. Verbindungen nach Anspruch 1, wobei in der allgemeinen Formel (I) Ⓐ ein Phenyl-, Pyridin-, Pyrazin-, oder Dihydropyridinring ist.

3. Verbindungen nach Anspruch 1 oder 2, wobei in der allgemeinen Formel (I) Ⓐ eine Dihydropyridin- oder Pyridingruppe darstellt, gegebenenfalls substituiert mit einer Gruppe ausgewählt aus den Gruppen -CH₂-CO-OAlkyl, -CH₂-CO-OAlkyl-OH, -CH₂-O-Alkyl-OAc, und/oder gegebenenfalls in Form eines Pyridiniumhalogenid..

4. Verbindungen nach einem der vorhergehenden Ansprüche, wobei in der allgemeinen Formel (I) Ⓑ ein Phenylring ist, substituiert mit einer (C₅-C₂₀)Alkyl-, O(C₅-C₂₀)Alkyl-, -S(O)ₚAlkyl-Gruppe, wobei p = 0, 1 oder 2 ist.

5. Verbindungen nach einem der vorhergehenden Ansprüche, wobei in der allgemeinen Formel (I) Ⓑ substituiert in ortho- oder meta-Position ist.

6. Verbindungen nach einem der vorhergehenden Ansprüche, wobei in der allgemeinen Formel (I) R3 = OH oder -Oalkyl und R1 und R2 zusammen eine Einfachbindung bilden.

7. Verbindungen nach einem der Ansprüche 1 bis 5, wobei in der allgemeinen Formel (I) R3 und R2 eine =O-Gruppe bilden und R1 ein Wasserstoffatom darstellt.

8. Verbindungen nach einem der vorhergehenden Ansprüche, wobei in der allgemeinen Formel (I):
Ⓐ eine Dihydropyridin-Gruppe gegebenenfalls substituiert mit einer Gruppe ausgewählt aus -CH₂COOAlkyl, -CH₂COOAlkylOH darstellt;
Ⓑ eine Phenylgruppe substituiert in meta-Position mit einer Gruppe ausgewählt aus den Gruppen C₅-C₂₀ Alkyl, -O(C₂-C₂₀) Alkyl darstellt; R1 und R2 zusammen eine Einfachbindung bilden, und R3 = OH, oder R1 = H und R2 und R3 zusammen eine =O-Gruppe bilden,
im Zustand einer Base oder eines Säureadditionssalzes, sowie im Zustand eines Hydrates oder eines Solvates, welche pharmazeutisch verträglich sind.

9. Verbindungen nach Anspruch 1, wobei die gegebenenfalls substituierte Tetrahydrofuran-Gruppe 2-Hydroxymethyl-Tetrahydrofuran-3,4-Diol ist.

10. Verbindungen nach Anspruch 1, wobei das Kation des Pyridiniums Bromid ist.

11. Verbindungen nach einem der vorhergehenden Ansprüche ausgewählt aus den folgenden Verbindungen:
- 1-(3-Dodecylphenyl)-1-Hydroxy-1,2-Dihydropyrrolo[3,4-*c*]pyridin-3-on;
- 1-(3-Dodecyloxyphenyl)-1-Hydroxy-1,2-Dihydropyrrolo[3,4-*c*]pyridin-3-on;
- 1-(3-Dodecylphenyl)-5-[2-(Ethyloxy)-2-Oxoethyl]-1-Hydroxy-3-Oxo-1,2-Dihydropyrrolo[3,4-c]pyridinium-Bromid;
- 3-Aminocarbonyl-[4-(3-Dodecylbenzoyl)-1,4-Dihydro-Pyudin-1-yl] Ethylacetat;
- 1-(3-Octyloxyphenyl)-1-Hydroxy-1,2-Dihydro-3*H*-Pyrrolo[3,4-*c*]pyridin-3-on;
- 1-(2-Dodecyloxyphenyl)-1-Hydroxy-1,2-Dihydro-3*H*-Pyrrolo[3,4-*c*]pyridin-3-on;
- 1-(3-Octodecyloxyphenyl)-1-Hydroxy-1,2-Dihydro-3*H*-Pyrrolo[3,4-*c*]pyridin-3-on;
- 1-[3-(Dodecylthio)phenyl]-1-Hydroxy-1,2-Dihydro-3*H*-Pyrrolo[3,4-*c*]pyridin-3-on;
- 1-(3-Dodecyloxyphenyl)-1-Methoxy-1,2-Dihydro-3*H*-Pyrrolo[3,4-*c*]pyridin-3-on;
- 1-Hydroxy-1-(3-(Propen-3-yl)Oxyphenyl)-1,2-Dihydropyrrolo[3,4-*c*]pyridin-3-on;
- 1-Hydroxy-1-(3-Hydroxyphenyl)-1,2-Dihydropyrrolo[3,4-*c*]pyridin-3-on;
- 1-Hydroxy-1-(3-Octylphenyl)-1,2-Dihydropyrrolo[3,4-*r*]pyridin-3-on;
- 1-Hydroxy-1-(3-(4-Nonylphenyl)phenyl)-1,2-Dihydropyrrolo[3,4-*c*]pyridin-3-on;
- 1-[3-(Dodecyloxy)phenyl]-5-(2-Ethoxy-2-Oxoethyl)-1-Hydroxy-3- Oxo-2,3-Dihydro-1*H*-Pyrrolo[3,4-*c*]pyridin-5-ium-Bromid;
- 1-[(3-(Dodecyloxy)phenyl)-1-Hydroxy-5-[2-(3-Hydroxypropoxy)-2-Oxyethyl]-3-Oxo-2,3-Dihydro-1*H*-Pyrrolo[3,4-*c*]pyridin-5-ium-Bromid;
- 3-Aminocarbonyl[4-(3-Dodécyloxybenzoyl)-1,4-Dihydropyridin-1-yl]Acetat;
- 3-Aminocarbonyl [4-(3-Dodécyloxybenzoyl)-1,4-Dihydropyridin-1-yl]Acetat 3-Hydroxypropyl;
im Zustand einer Base oder eines Säureadditionssalzes, sowie im Zustand eines Hydrates oder eines Solvates, welche pharmazeutisch verträglich sind.

12. Verfahren zur Herstellung einer Verbindung der allgemeinen Formel (I) nach einem der vorhergehenden Ansprüche, wobei R1 und R2 zusammen eine Einfachbindung bilden und R3 -OH darstellt, aufweisend den Schritt des Kuppelns einer Verbindung der allgemeinen Formel (II): 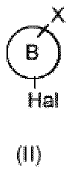 mit einer Verbindung der allgemeinen Formel (III): 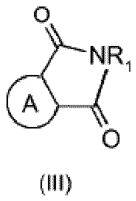 was zu der Verbindung der Formel (IV) führt: 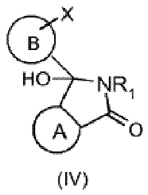 wobei Ⓐ, Ⓑ und R1 wie in der allgemeinen Formel (I) definiert sind, Hal ein Halogenatom darstellt und X entweder ein Wasserstoffatom, wenn Ⓑ nicht substituiert ist, oder den Substituenten des Ⓑ, welcher der begehrten allgemeinen Formel (I) entspricht (in diesen beiden Fällen die Verbindung (IV) entspricht der Verbindung (I)) oder ein Halogenatom darstellt, wobei die Kupplung in diesem Fall von der Subsitutionsreaktion des Halogenatoms der Verbindung (IV) mit dem angemessenen Substituenten des Ⓑ gefolgt ist, welcher der begehrten allgemeinen Formel (I) entspricht.

13. Verfahren zur Herstellung einer Verbindung der allgemeinen Formel (I) nach einem der Ansprüche 1 bis 9, wobei R1 und R2 zusammen eine Einfachverbindung bilden und R3 -OH darstellt, aufweisend einen Schritt des Cyclisierens der Verbindungen der allgemeinen Formel (V) 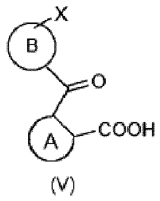 wobei Ⓐ, Ⓑ und Hal wie in der allgemeinen Formel (IV) in Gegenwart einer NHR1 (V') definiert sind,
was zu der Verbindung der Formel (IV) führt: 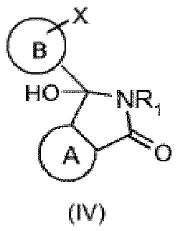 wobei R1 wie in der allgemeinen Formel (I) definiert ist, und X entweder ein Wasserstoffatom, wenn Ⓑ nicht substituiert ist, oder den Substituenten des Ⓑ, welcher der begehrten allgemeinen Formel (I) entspricht (in diesen beiden Fällen die Verbindung (IV) entspricht der Verbindung (I)) oder ein Halogenatom darstellt, wobei die Cyclisierung dann in diesem Fall von der Subsitutionsreaktion des Halogenatoms der Verbindung (IV) mit dem angemessenen Substituenten des Ⓑ gefolgt ist, welcher der begehrten allgemeinen Formel (I) entspricht.

14. Verfahren nach Anspruch 12 oder 13 zur Herstellung einer Verbindung der allgemeinen Formel (I), wobei Ⓐ einen Pyridinium-Ring darstellt, in welchem das Stickstoffatom quatemisiert ist, wobei das Verfahren zur Herstellung zusätzlich einen nachfolgenden Schritt aufweist, welcher darin besteht, die Verbindung der allgemeinen Formel (I) zu quaternisieren, bei der Ⓐ einen Pyridin-Ring darstellt, mittels einer Verbindung der allgemeinen Formel (XII):
R-Hal (XII)
wobei Hal ein Halogenatom darstellt und R eine Gruppe des Typs -CH₂-E darstellt, wobei E eine elektronenziehende Gruppe wie in Anspruch 1 definiert ist.

15. Verfahren zur Herstellung einer Verbindung der allgemeinen Formel (I) nach
einem der Ansprüche 1 bis 11, wobei Ⓐ einen Dihydropyridin-Ring darstellt und R2 und R3 zusammen eine =O-Gruppe bilden und R1 ein Wasserstoffatom darstellt, wobei das Verfahren das Verfahren nach Anspruch 12 darstellt, welches von dem Schritt des Reduzierens der Verbindung der allgemeinen Formel (I) gefolgt ist, in welchem Ⓐ einen Pyridinium-Ring darstellt.

16. Verfahren nach einem der Ansprüche 12 bis 15 aufweisend zusätzlich den Schritt des Isolierens und/oder des Reinigens des erhaltenen Produkts.

17. Pharmazeutische Zusammensetzung aufweisend als Wirkstoff eine Verbindung der allgemeinen Formel (I) wie in einem der Ansprüche 1 bis 11 definiert
und mindestens einen pharmazeutisch verträglichen Träger.

18. Kombination einer Verbindung der allgemeinen Formel (I) nach einem der Ansprüche 1 bis 11 mit einem Wirkstoff mit antiinfektiöser Aktivität.

19. Verwendung einer Verbindung nach einem der Ansprüche 1 bis 11 zur Herstellung eines antiinfektiösen Medikaments.

20. Verbindung der allgemeinen Formel nach einem der Ansprüche 1 bis 11 zur Verwendung in der Behandlung und/oder Vorbeugung von Infektionen.

21. Verbindung zur Verwendung nach Anspruch 20, wobei die menschlichen oder tierischen Infektionen ausgewählt sind aus mycobakteriellen Krankheiten, Lepra oder opportunistischen Krankheiten, wie diejenigen, die mit *Mycobacterium avium*, *M. bovis* und/oder *M. ulcerans, M. marinum* verbunden sind; Malaria oder jeglicher Infektion, welche mit einem Krankheitserreger verbunden sind, welcher ein Enzym des Typs Enoyl-Acyl-Trägerprotein-Reduktase (enoyl-Acyl Carrier Protein reductase) oder ein strukturell verwandtes Enzym aufweist, welches zu der strukturellen Superfamilie der Kurzkettigen Dehydrogenasen Reduktasen (SDR) gehören.

22. Verbindung zur Verwendung nach Anspruch 21, wobei die mycobakteriellen Krankheiten ausgewählt sind aus Tuberkulose, Lepra oder opportunistischen Krankheiten, wie diejenigen, die mit *Mycobacterium avium*, *M. bovis* und/oder *M. ulcerans, M. marinum* verbunden sind.

23. Verbindung zur Verwendung nach Anspruch 21 oder 22 zur Verwendung in der Behandlung und/oder Vorbeugung von Tuberkulose.

## Claims

1. Compounds of general formula (I): 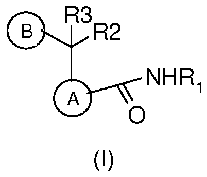 in which:
- the cycle Ⓐ represents a 6-membered aromatic or non-aromatic ring, optionally comprising one or more nitrogen atoms, said nitrogen atom(s) optionally being substituted by
an optionally substituted tetrahydrofuran group,or by
a -CH₂-E group, wherein E is chosen from -CONRR'; -CO-SR; -CO-Oalkyl; -CO-Oalkyl-OH; -O-alkyl-OAc; phenyl substituted for example by a CN or NO₂ group; wherein R and R' are the same or different and independently represent a hydrogen atom or an alkyl group;
and/or wherein said nitrogen atom(s) may be in the form of pyridinium salts, the counter ion being the anion of a halogen atom;
- Ⓑ represents a 5-to-10-membered mono- or bicyclic aryl or heteroaryl group, substituted by one or more groups selected from the groups -(C₅-C₂₀)alkyl; -O(C₂-C₂₀)alkyl; -S(O)ₚalkyl wherein p = 0, 1 or 2 ; alkenyl; alkynyl;
- R1 represents a hydrogen atom or an alkyl group and R2 and R3 together form an =O group;
or alternatively
R3 represents an -OH or -Oalkyl group and R2 forms, together with R1, a single bond binding the nitrogen atom to the carbon atom substituted by R3, Ⓐ and Ⓑ, in such a way as to form an indanone ring by fusion with the cycle Ⓐ;
the groups -C(=O)NHR1 and C(R3)(R2)- being understood to be located in two adjacent positions on the ring Ⓐ;
in the form of a base or an acid addition salt, as well as in the form of a pharmaceutically acceptable hydrate or solvate.

2. Compounds according to claim 1, wherein in the general formula (I), Ⓐ is a phenyl, pyridine, pyrazine or dihydropyridine ring.

3. Compounds according to either claim 1 or claim 2, wherein in the general formula (I), Ⓐ represents a dihydropyridine or pyridine group, optionally substituted by a group selected from the groups -CH₂-CO-Oalkyl; -CH₂-CO-Oalkyl-OH; -CH₂-O-alkyl-OAc, and/or optionally in the form of a pyridinium halide.

4. Compounds according to any one of the preceding claims, wherein in the general formula (I), Ⓑ is a phenyl ring substituted by a (C₅-C₂₀)alkyl, , O(C₅-C₂₀)alkyl or - S(O)ₚalkyl group wherein p = 0, 1 or 2.

5. Compounds according to any one of the preceding claims, wherein in the general formula (I), Ⓑ is substituted in the ortho or meta position.

6. Compounds according to any one of the preceding claims, wherein in the general formula (I), R3 = OH or -Oalkyl and R1 and R2 together form a single bond.

7. Compounds according to any one of claims 1 to 5, wherein in the general formula (I), R3 and R2 form an =O group and R1 represents a hydrogen atom.

8. Compounds according to any one of the preceding claims, wherein in the general formula (I):
Ⓐ represents a dihydropyridine group, optionally substituted by a group selected from -CH₂COOalkyl, -CH₂COOalkylOH;
Ⓑ represents a phenyl group, substituted in the meta position, by a group selected from the groups C₅-C₂₀ alkyl, -O(C2-C20)alkyl, R1 and R2 together form a single bond and R3 = OH, or R1 = H and R2 and R3 together form an =O group;
in the form of a base or an acid addition salt, as well as in the form of a pharmaceutically acceptable hydrate or solvate.

9. Compounds according to claim 1 where the optionally substituted tetrahydrofuran group is 2-hydroxymethyl-tetrahydrofuran-3,4-diol.

10. Compounds according to claim 1 where the pyridinium cation is bromide.

11. Compounds according to any one of the preceding claims, selected from the following compounds:
- 1-(3-dodecylphenyl)-1-hydroxy-1,2-dihydropyrrolo[3,4-c]pyridin-3-one;
- 1-(3-dodecyloxyphenyl)-1-hydroxy-1,2-dihydropyrrolo[3,4-c]pyridin-3-one;
- 1-(3-dodecylphenyl)-5-[2-(ethyloxy)-2-oxoethyl]-1-hydroxy-3-oxo-1,2-dihydropyrrolo[3,4-c]pyridinium bromide;
- ethyl 3-aminocarbonyl-[4-(3-dodecylbenzoyl)-1,4-dihydro-pyridin-1-yl] acetate;
- 1-(3-octyloxyphenyl)-1-hydroxy-1,2-dihydro-3*H*-pyrrolo[3,4-c]pyridin-3-one;
- 1-(2-dodecyloxyphenyl)-1-hydroxy-1,2-dihydro-3*H*-pyrrolo[3,4-*c*]pyridin-3-one;
- 1-(3-octodecyloxyphenyl)-1-hydroxy-1,2-dihydro-3*H*-pyrrolo[3,4-*c*]pyridin-3-one;
- 1-[3-(dodecylthio)phenyl]-1-hydroxy-1,2-dihydro-3*H*-pyrrolo[3,4-*c*]pyridin-3-one;
- 1-(3-dodecyloxyphenyl)-1-methoxy-1,2-dihydro-3*H*-pyrrolo[3,4-*c*]pyridin-3-one;
- 1-hydroxy-1-(3-(propen-3-yl)oxyphenyl)-1,2-dihydropyrrolo[3,4-c]pyridin-3-one;
- 1-hydroxy-1-(3-octylphenyl)-1,2-dihydropyrrolo[3,4-*c*]pyridin-3-one;
- 1-hydroxy-1-(3-(4-nonylphenyl)phenyl)-1,2-dihydropyrrolo[3,4-*c*]pyridin-3-one;
- 1-[3-(dodecyloxy)phenyl]-5-(2-ethoxy-2-oxoethyl)-1-hydroxy-3-oxo-2,3-dihydro-1*H-*pyrrolo[3,4-*c*]pyridin-5-ium bromide;
- 1-[(3-(dodecyloxy)phenyl)-1-hydroxy-5-[2-(3-hydroxypropoxy)-2-oxyethyl]-3-oxo-2,3-dihydro-1*H*-pyrrolo[3,4-*c*]pyridin-5-ium bromide;
- ethyl 3-aminocarbonyl-[4-(3-dodecyloxybenzoyl)-1,4-dihydropyridin-1-yl]acetate;
- 3-hydroxypropyl 3-aminocarbonyl-[4-(3-dodecyloxybenzoyl)-1,4-dihydropyridin-1-yl]acetate;
in the form of a base or an acid addition salt, as well as in the form of a pharmaceutically acceptable hydrate or solvate.

12. Method for the preparation of a compound of general formula (I) according to any of the preceding claims in which R1 and R2 together form a single bond and R3 represents -OH, comprising the step of coupling a compound of general formula (II): 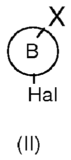 and a compound of general formula (III): 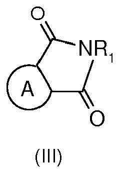 resulting in a compound of formula (IV) : 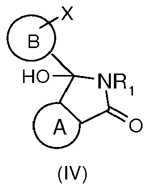 in which Ⓐ, Ⓑ and R1 are defined as in general formula (I), Hal represents a halogen atom and X represents a hydrogen atom, when Ⓑ is not substituted, or the substituent of Ⓑ corresponding to the desired general formula (I) (in these two cases, the compound (IV) corresponds to the compound (I)), or a halogen atom, the coupling being followed in this case by the reaction substituting the halogen atom of the compound (IV) with the appropriate substituent of Ⓑ corresponding to the desired general formula (I).

13. Method for the preparation of a compound of general formula (I) according to any one of claims 1 to 9 in which R1 and R2 together form a single bond and R3 represents -OH, comprising the step of cyclising compounds of general formula (V): 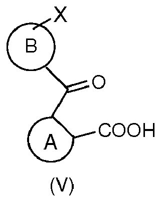 in which Ⓐ, Ⓑ and Hal are defined as in general formula (IV) in the presence of NHR1 (V'),
resulting in the compound of formula (IV): 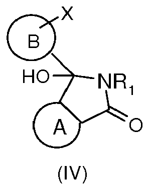 in which R1 is defined as in general formula (I) and X represents a hydrogen atom, when Ⓑ is not substituted, or the substituent of Ⓑ corresponding to the desired general formula (I) (in these two cases, the compound (IV) corresponds to the compound (I)), or a halogen atom, the cyclisation being followed in this case by the reaction substituting the halogen atom of the compound (IV) with the appropriate substituent of Ⓑ corresponding to the desired general formula (I).

14. Method according to either claim 12 or claim 13 for the preparation of a compound of general formula (I) in which Ⓐ represents a pyridinium ring in which the nitrogen atom is quaternised, the preparation method further comprising the subsequent step of quaternising the compound of general formula (I) in which Ⓐ represents a pyridine ring using a compound of general formula (XII):
R-Hal (XII)
in which Hal represents a halogen atom and R represents a group of the -CH₂E type, E being an electron-attracting group as defined in claim 1.

15. Method for the preparation of a compound of general formula (I) according to anyone of claims 1 to 11, in which Ⓐ represents a dihydropyridine ring and R2 and R3 together form an =O group and R1 represents a hydrogen atom, said method comprising the method according to claim 12 followed by the step of reducing the compound of general formula (I) in which Ⓐ represents a pyridinium ring.

16. Method according to any one of claims 12 to 15, further comprising the reaction to isolate and/or purify the obtained product.

17. Pharmaceutical composition comprising as an active ingredient a compound of general formula (I):
as defined in anyone of claims 1 to 11
and at least one pharmaceutically acceptable excipient.

18. Combination of a compound of general formula (I) according to any one of claims 1 to 11 and an anti-infective active ingredient.

19. Use of a compound according to any one of claims 1 to 11 for the preparation of an anti-infective drug.

20. Compound of general formula (I) according to any one of claims 1 to 11 for its use for the treatment and/or prevention of infections.

21. Compound for use according to claim 20, wherein said human or animal infections are selected from mycobacterioses, leprosy or opportunistic diseases, such as those caused by *Mycobacterium avium, M. bovis* and/or *M*. *ulcerans, M. marinum*; malaria, or any infection caused by a pathogen having an enzyme of the enoyl acyl carrier protein reductase type or an enzyme of a related structure belonging to the short chain dehydrogenase reductase (SDR) superfamily.

22. Compound for use according to claim 21, wherein the mycobacterioses are selected from tuberculosis, leprosy or opportunistic diseases, such as those caused by *Mycobacterium avium, M. bovis* and/or *M*. *ulcerans, M. marinum*

23. Compound according to claim 21 or 22 for its use for the treatment and/or prevention of tuberculosis.
